# EUROPEAN PATENT APPLICATION

(11) **EP 1 354 891 A1**
(43) Date of publication of application: **22.10.2003**
(21) Application number: 03003693.3
(22) Date of filing: 18.02.2003
(51) Int. Cl.: C07K 14/475, A61K 38/18, A61K 48/00

(54) **Medicament comprising NK4 gene or recombinant NK4 protein**

(30) Priority: 25.02.2002 JP 2002048644
(71) Applicant: Kringle Pharma Inc., Osaka 541-0047 (JP); NAKAMURA, Toshikazu, Takatsukishi, Osaka 569-1020 (JP)
(72) Inventor: Nakamura, Toshikazu, Takatsuki-shi, Osaka 569-1020 (JP); Matsumoto, Kunio, Mino-shi, Osaka 562-0031 (JP)
(74) Representative: Keller, Günter, Dr.

(57) **Abstract**

The present invention provides a therapeutic agent which is effective for primary tumor of cancer and, more particularly, it provides an NK4 gene therapeutic agent and a recombinant NK4 protein preparation which is effective for the prevention and therapy of cancer. The present invention also provides a therapeutic agent which is effective for metastasis of cancer and, more particularly, it provides an NK4 gene therapeutic agent and a recombinant NK4 protein preparation which is effective for the prevention and therapy of metastasis of cancer.

The present invention further provides a therapeutic agent which is effective for diseases caused by neovascularization including cancer and, more particularly, it provides an NK4 gene therapeutic agent and a recombinant NK4 protein preparation which is effective for diseases caused by neovascularization.

(Solving Means)

DNA containing a base sequence represented by SEQ ID NO: 1 or SEQ ID NO: 2 or DNA containing a DNA which hybridizes to the said DNA under a stringent condition.

SEQUENCE LISTING

<110> Kringle Pharma Inc.

<110> NAKAMURA Toshikazu

<120> Medicament comprising NK4 gene or recombinant NK4 protein

<130> DK12F774(EP)P

<150> JP 2002-048644

<151> 2002-2-25

<160> 2

<210> 1

<211> 1341

<212> DNA

<213> Homo sapiens

<400> 1

caa agg aaa aga aga aat aca att cat gaa ttc aaa aaa tca gca aag 48

Gln Arg Lys Arg Arg Asn Thr Ile His Glu Phe Lys Lys Ser Ala Lys

1               5                   10                  15

act acc cta atc aaa ata gat cca gca ctg aag ata aaa acc aaa aaa 96

Thr Thr Leu Ile Lys Ile Asp Pro Ala Leu Lys Ile Lys Thr Lys Lys

            20                  25                  30

gtg aat act gca gac caa tgt gct aat aga tgt act agg aat aaa gga 144

Val Asn Thr Ala Asp Gln Cys Ala Asn Arg Cys Thr Arg Asn Lys Gly

        35                  40                  45

ctt cca ttc act tgc aag gct ttt gtt ttt gat aaa gca aga aaa caa 192

Leu Pro Phe Thr Cys Lys Ala Phe Val Phe Asp Lys Ala Arg Lys Gln

    50                  55                  60

tgc ctc tgg ttc ccc ttc aat agc atg tca agt gga gtg aaa aaa gaa 240

Cys Leu Trp Phe Pro Phe Asn Ser Met Ser Ser Gly Val Lys Lys Glu

65                  70                  75                  80

tttggc cat gaa ttt gac ctc tat gaa aac aaa gac tac att aga aac 288

Phe Gly His Glu Phe Asp Leu Tyr Glu Asn Lys Asp Tyr Ile Arg Asn

                85                  90                  95

tgc atc att ggt aaa gga cgc agc tac aag gga aca gta tct atc act 336

Cys Ile Ile Gly Lys Gly Arg Ser Tyr Lys Gly Thr Val Ser Ile Thr

            100                 105                 110

aag agt ggc atc aaa tgt cag ccc tgg agt tcc atg ata cca cac gaa 384

Lys Ser Gly Ile Lys Cys Gln Pro Trp Ser Ser Met Ile Pro His Glu

        115                 120                 125

cac agc ttt ttg cct tcg agc tat cgg ggt aaa gac cta cag gaa aac 432

His Ser Phe Leu Pro Ser Ser Tyr Arg Gly Lys Asp Leu Gln Glu Asn

    130                 135                 140

tac tgt cga aat cct cga ggg gaa gaa ggg gga ccc tgg tgt ttc aca 480

Tyr Cys Arg Asn Pro Arg Gly Glu Glu Gly Gly Pro Trp Cys Phe Thr

145                 150                 155                 160

agc aat cca gag gta cgc tac gaa gtc tgt gac att cct cag tgt tca 528

Ser Asn Pro Glu Val Arg Tyr Glu Val Cys Asp Ile Pro Gln Cys Ser

                165                 170                 175

gaa gtt gaa tgc atg acc tgc aat ggg gag agt tat cga ggt ctc atg 576

Glu Val Glu Cys Met Thr Cys Asn Gly Glu Ser Tyr Arg Gly Leu Met

            180                 185                 190

gat cat aca gaa tca ggc aag att tgt cag cgc tgg gat cat cag aca 624

Asp His Thr Glu Ser Gly Lys Ile Cys Gln Arg Trp Asp His Gln Thr

        195                 200                 205

cca cac cgg cac aaa ttc ttg cct gaa aga tat ccc gac aag ggc ttt 672

Pro His Arg His Lys Phe Leu Pro Glu Arg Tyr Pro Asp Lys Gly Phe

    210                 215                 220

gat gat aat tat tgc cgc aat ccc gat ggc cag ccg agg cca tgg tgc 720

Asp Asp Asn Tyr Cys Arg Asn Pro Asp Gly Gln Pro Arg Pro Trp Cys

225                 230                 235                 240

tat act ctt gac cct cac acc cgc tgg gag tac tgt gca att aaa aca  768

Tyr Thr Leu Asp Pro His Thr Arg Trp Glu Tyr Cys Ala Ile Lys Thr

                245                 250                 255

tgc gct gac aat act atg aat gac act gat gtt cct ttg gaa aca act  816

Cys Ala Asp Asn Thr Met Asn Asp Thr Asp Val Pro Leu Glu Thr Thr

            260                 265                 270

gaa tgc atc caa ggt caa gga gaa ggc tac agg ggc act gtc aat acc  864

Glu Cys Ile Gln Gly Gln Gly Glu Gly Tyr Arg Gly Thr Val Asn Thr

        275                 280                 285

att tgg aat gga att cca tgt cag cgt tgg gat tct cag tat cct cac  912

Ile Trp Asn Gly Ile Pro Cys Gln Arg Trp Asp Ser Gln Tyr Pro His

    290                 295                 300

gag cat gac atg act cct gaa aat ttc aag tgc aag gac cta cga gaa  960

Glu His Asp Met Thr Pro Glu Asn Phe Lys Cys Lys Asp Leu Arg Glu

305                 310                 315                 320

aat tac tgc cga aat cca gat ggg tct gaa tca ccc tgg tgt ttt acc 1008

Asn Tyr Cys Arg Asn Pro Asp Gly Ser Glu Ser Pro Trp Cys Phe Thr

                325                 330                 335

act gat cca aac atc cga gtt ggc tac tgc tcc caa att cca aac tgt 1056

Thr Asp Pro Asn Ile Arg Val Gly Tyr Cys Ser Gln Ile Pro Asn Cys

            340                 345                 350

gat atg tca cat gga caa gat tgt tat cgt ggg aat ggc aaa aat tat 1104

Asp Met Ser His Gly Gln Asp Cys Tyr Arg Gly Asn Gly Lys Asn Tyr

        355                 360                 365

atg ggc aac tta tcc caa aca aga tct gga cta aca tgt tca atg tgg 1152

Met Gly Asn Leu Ser Gln Thr Arg Ser Gly Leu Thr Cys Ser Met Trp

    370                 375                 380

gac aag aac atg gaa gac tta cat cgt cat atc ttc tgg gaa cca gat 1200

Asp Lys Asn Met Glu Asp Leu His Arg His Ile Phe Trp Glu Pro Asp
385                 390                 395                 400
gca agt aag ctg aat gag aat tac tgc cga aat cca gat gat gat gct 1248
Ala Ser Lys Leu Asn Glu Asn Tyr Cys Arg Asn Pro Asp Asp Asp Ala
                405                 410                 415
cat gga ccc tgg tgc tac acg gga aat cca ctc att cct tgg gat tat 1296
His Gly Pro Trp Cys Tyr Thr Gly Asn Pro Leu Ile Pro Trp Asp Tyr
            420                 425                 430
tgc cct att tct cgt tgt gaa ggt gat acc aca cct aca ata gtc    1341
Cys Pro Ile Ser Arg Cys Glu Gly Asp Thr Thr Pro Thr Ile Val
        435                 440                 445

<210> 2
<211> 1326
<212> DNA
<213> Homo sapiens
<400> 2
caa agg aaa aga aga aat aca att cat gaa ttc aaa aaa tca gca aag   48
Gln Arg Lys Arg Arg Asn Thr Ile His Glu Phe Lys Lys Ser Ala Lys
 1               5                  10                  15
act acc cta atc aaa ata gat cca gca ctg aag ata aaa acc aaa aaa   96
Thr Thr Leu Ile Lys Ile Asp Pro Ala Leu Lys Ile Lys Thr Lys Lys
            20                  25                  30
gtg aat act gca gac caa tgt gct aat aga tgt act agg aat aaa gga  144
Val Asn Thr Ala Asp Gln Cys Ala Asn Arg Cys Thr Arg Asn Lys Gly
        35                  40                  45
ctt cca ttc act tgc aag gct ttt gtt ttt gat aaa gca aga aaa caa  192
Leu Pro Phe Thr Cys Lys Ala Phe Val Phe Asp Lys Ala Arg Lys Gln
    50                  55                  60

tgc ctc tgg ttc ccc ttc aat agc atg tca agt gga gtg aaa aaa gaa 240
Cys Leu Trp Phe Pro Phe Asn Ser Met Ser Ser Gly Val Lys Lys Glu
65                  70                  75                  80
ttt ggc cat gaa ttt gac ctc tat gaa aac aaa gac tac att aga aac 288
Phe Gly His Glu Phe Asp Leu Tyr Glu Asn Lys Asp Tyr Ile Arg Asn
                85                  90                  95
tgc atc att ggt aaa gga cgc agc tac aag gga aca gta tct atc act 336
Cys Ile Ile Gly Lys Gly Arg Ser Tyr Lys Gly Thr Val Ser Ile Thr
            100                 105                 110
aag agt ggc atc aaa tgt cag ccc tgg agt tcc atg ata cca cac gaa 384
Lys Ser Gly Ile Lys Cys Gln Pro Trp Ser Ser Met Ile Pro His Glu
        115                 120                 125
cac agc tat cgg ggt aaa gac cta cag gaa aac tac tgt cga aat cct 432
His Ser Tyr Arg Gly Lys Asp Leu Gln Glu Asn Tyr Cys Arg Asn Pro
    130                 135                 140
cga ggg gaa gaa ggg gga ccc tgg tgt ttc aca agc aat cca gag gta 480
Arg Gly Glu Glu Gly Gly Pro Trp Cys Phe Thr Ser Asn Pro Glu Val
145                 150                 155                 160
cgc tac gaa gtc tgt gac att cct cag tgt tca gaa gtt gaa tgc atg 528
Arg Tyr Glu Val Cys Asp Ile Pro Gln Cys Ser Glu Val Glu Cys Met
                165                 170                 175
acc tgc aat ggg gag agt tat cga ggt ctc atg gat cat aca gaa tca 576
Thr Cys Asn Gly Glu Ser Tyr Arg Gly Leu Met Asp His Thr Glu Ser
            180                 185                 190
ggc aag att tgt cag cgc tgg gat cat cag aca cca cac cgg cac aaa 624
Gly Lys Ile Cys Gln Arg Trp Asp His Gln Thr Pro His Arg His Lys
        195                 200                 205
ttc ttg cct gaa aga tat ccc gac aag ggc ttt gat gat aat tat tgc 672
Phe Leu Pro Glu Arg Tyr Pro Asp Lys Gly Phe Asp Asp Asn Tyr Cys

210                 215                 220

cgc aat ccc gat ggc cag ccg agg cca tgg tgc tat act ctt gac cct  720

Arg Asn Pro Asp Gly Gln Pro Arg Pro Trp Cys Tyr Thr Leu Asp Pro

225                 230                 235                 240

cac acc cgc tgg gag tac tgt gca att aaa aca tgc gct gac aat act  768

His Thr Arg Trp Glu Tyr Cys Ala Ile Lys Thr Cys Ala Asp Asn Thr

                245                 250                 255

atg aat gac act gat gtt cct ttg gaa aca act gaa tgc atc caa ggt  816

Met Asn Asp Thr Asp Val Pro Leu Glu Thr Thr Glu Cys Ile Gln Gly

            260                 265                 270

caa gga gaa ggc tac agg ggc act gtc aat acc att tgg aat gga att  864

Gln Gly Glu Gly Tyr Arg Gly Thr Val Asn Thr Ile Trp Asn Gly Ile

        275                 280                 285

cca tgt cag cgt tgg gat tct cag tat cct cac gag cat gac atg act  912

Pro Cys Gln Arg Trp Asp Ser Gln Tyr Pro His Glu His Asp Met Thr

    290                 295                 300

cct gaa aat ttc aag tgc aag gac cta cga gaa aat tac tgc cga aat  960

Pro Glu Asn Phe Lys Cys Lys Asp Leu Arg Glu Asn Tyr Cys Arg Asn

305                 310                 315                 320

cca gat ggg tct gaa tca ccc tgg tgt ttt acc act gat cca aac atc 1008

Pro Asp Gly Ser Glu Ser Pro Trp Cys Phe Thr Thr Asp Pro Asn Ile

                325                 330                 335

cga gtt ggc tac tgc tcc caa att cca aac tgt gat atg tca cat gga 1056

Arg Val Gly Tyr Cys Ser Gln Ile Pro Asn Cys Asp Met Ser His Gly

            340                 345                 350

caa gat tgt tat cgt ggg aat ggc aaa aat tat atg ggc aac tta tcc 1104

Gln Asp Cys Tyr Arg Gly Asn Gly Lys Asn Tyr Met Gly Asn Leu Ser

        355                 360                 365

caa aca aga tct gga cta aca tgt tca atg tgg gac aag aac atg gaa 1152

Gln Thr Arg Ser Gly Leu Thr Cys Ser Met Trp Asp Lys Asn Met Glu
    370                 375                 380
gac tta cat cgt cat atc ttc tgg gaa cca gat gca agt aag ctg aat 1200
Asp Leu His Arg His Ile Phe Trp Glu Pro Asp Ala Ser Lys Leu Asn
385                 390                 395                 400
gag aat tac tgc cga aat cca gat gat gat gct cat gga ccc tgg tgc 1248
Glu Asn Tyr Cys Arg Asn Pro Asp Asp Asp Ala His Gly Pro Trp Cys
                405                 410                 415
tac acg gga aat cca ctc att cct tgg gat tat tgc cct att tct cgt 1296
Tyr Thr Gly Asn Pro Leu Ile Pro Trp Asp Tyr Cys Pro Ile Ser Arg
            420                 425                 430
tgt gaa ggt gat acc aca cct aca ata gtc 1326
Cys Glu Gly Asp Thr Thr Pro Thr Ile Val
        435                 440

## Description

### BACKGROUND OF THE INVENTION

### 1. Field of the Invention

The present invention relates to an agent for gene therapy for the prevention and/or therapy of cancer and/or the diseases caused by neovascularization utilizing the DNA which codes for NK4. More particularly, it relates to an agent for gene therapy and a method for gene therapy in which DNA coding for NK4 is introduced into cells of organism and/or into organism so as to suppress tumor invasion, growth and metastasis, to induce apoptosis and/or to suppress neovascularization.

The present invention further relates to an art where recombinant expression vector which contains DNA coding for NK4 utilizing a gene recombination technique, transformant which is transformed by the said recombinant expression vector and genetically recombined NK4 which is useful as anti-cancer agent and/or neovascularization suppressor are provided in large quantities and also in an economical manner.

The present invention furthermore relates to a recombinant NK4 protein preparation for the prevention and/or therapy of cancer and/or diseases caused by neovascularization. More particularly, it relates to a recombinant NK4 protein preparation and method of therapy to suppress tumor invasion, growth and metastasis, to induce apoptosis and/or to suppress neovascularization.

### 2. Description of the Related Art

For the therapy of cancer, there has been carried out surgical therapy, chemotherapy, radiotherapy or a multidisciplinary therapy which is a combination thereof etc.

On the other hand, many people who pass away of cancer die not of primary tumor but of metastases of neoplastic cells dissociated and spread from the primary tumor to other organs.

Thus, although it is possible to remove the tumor by surgical therapy if the primary tumor is able to be found in early stage, there are many cases in cancer such as pancreatic cancer and lung cancer which is apt to be metastasized that, even if the size of the primary tumor is small, metastatic tumor which is so small that being unable to be noticed by naked eye is formed when it is detected whereby it is difficult to surgically remove that. On the other hand, in chemotherapy using an anti-cancer agent or radiotherapy, its intended strategy is to directly kill the tumor cells and, therefore, even when the primary tumor temporarily becomes smaller by such a treatment, it is difficult to prevent the relapse of resistant cancer and the metastasis of surviving cancer. Further, anti-cancer agent and radiotherapy kill not only the cancer cells but also normal cells and, as a result, a severe side effect is resulted whereby quality of life and immune strength of the patient are lowered.

As such, the current status is that there is no satisfactory therapeutic method for such a fatal metastasis of cancer and there has been a brisk demand for the development of medicaments which are effective for inhibition of cancer metastasis.

With regard to the mechanism of cancer metastasis, many studies have been carried out already. Carcinoma is mostly generated in epithelial tissues and carcinoma cells are liberated from primary tumor, break the basal membrane separating the epithelial tissue, invade into the surrounding tissues, invade into blood vessels and lymph gland, transferred to the distant tissues through blood or lymph vessels, invade again into the tissues together with neovascularization from blood and lymph vessels and grow whereupon metastasis is resulted.

For the development of cancer metastasis suppressors, it is believed that any of those processes is to be inhibited. Thus, there may be exemplified a substance which suppresses the adhesion to the endothelial cells of the metastasized area (Iwamoto, Y. et al., *Science,* 1132-1134 (1987)), a neovascularization inhibitor (Cao, Y. et al., *J. Clin. Invest.,* **101**, 1055-1063 (1988)), a substance which suppresses the invasion of cancer cells (Japanese Patent Laid-Open No. 03/31214) and an inhibitor for degrading enzyme of the basal membrane (Irimura, T., Nakajima, M. and Nicolson, G. L., *Biochemistry*, **25**, 5322-5328 (1989); Japanese Patent Laid-Open No. 05/194414).

Neovascularization is generated by the growth of capillary vessel of already-existing blood vessel. Neovascularization is essential for many physiological processes such as embryogenesis, curing of wound and regeneration of tissues or organs. On the other hand, in pathology such as tumor growth and intratumoral metastasis to distal sites, abnormal growth of neovascularization takes place. It has been known that initiation of the tumor neovascularization is induced by vascular endothelial growth factor (VEGF), basic fibroblast growth factor (bFGF), hepatocyte growth factor (HGF) and the like.

It has been known that HGF (hepatocyte growth factor) has mitogenic activity, motogenic activity, morphogenic activity and neovascularizing action and that, HGF mediates interaction between cancer cells and adjacent stromal cells (tumor-stroma interaction), thereby inducing invasion and metastasis of tumor (Nakamura, T., et al., *Cancer Res*., **57**, 3305-3313 (1997) ; Jiang, W. G., et al., *Crit. Rev. Oncol. Hematol*., **29**, 209-248 (1999)). HGF binds to the c-Met/HGF receptor and, as a result of tyrosine phosphorylation, invasion and metastasis of tumor are induced (Jiang, W. G., et al., *Crit. Rev. Oncol. Hematol*., **29**, 209-248 (1999)). It has been known that the c-Met/HGF receptor is often expressed excessively in cancer cells (Di. Renzo, et al., *Oncogene,* **6**, 1997-2003 (1991); Di. Renzo, et al., *Cancer Res.,* **55**, 1129-1138 (1995); Nakajima, M., et al., *Cancer,* **85**, 1894-1902 (1999)).

Recently, a tumor dormancy therapy has been receiving public attention as the therapy for cancer. The tumor dormancy therapy is that cancer cells are made into a dormant state using a neovascularization inhibitor. The neovascularization inhibitor does not directly kill the cancer cells but inhibits the neovascularization whereby the supplying route for nutrition and oxygen necessary for the growth of cancer cells are shut off, apoptosis is induced and the cancer cells are made into a dormant state. With regard to the neovascularization inhibitor, there have been known angiostatin (Cao, Y., et al., *J. Clin. Invest.,* **101,** 1055-1063 (1988)), endostatin (Blezinger, P., et al., *Nat. Biotechnol*., **17**, 343-348 (1999)) and the like.

NK4 has the N-terminal hairpin domain and subsequent four-Kringle domains of the α-chain of HGF, binds to a c-Met/HGF receptor and acts as an antagonist for HGF (Date, K., et al., *FEBS Lett.,* **420,** 1-6 (1997); Date, K. et al., *Oncogene,* **17**, 3045-3054 (1998)). It has been known that tumor invasion and metastasis are suppressed by the antagonistic activity of NK4 against HGF and that, neovascularization caused not only by HGF but also by VEGF and b FGF is suppressed by NK4 through a mechanism which is distinct from its antagonistic activity against HGF (Kuba, K., et al., *Cancer Res.,* **60,** 6737-6743 (2000)).

On the other hand, together with the progress of the technique for gene transfer into organism, a novel therapeutic field called gene therapy is now going to be established. Gene therapy with an object of cancer therapy has been abundantly carried out already and most of it is with an object that cancer cells are killed by introducing suicide gene into cancer cells. However, it is difficult to express the aimed gene in all cancer cells and, as same as in the case of chemotherapy and radiotherapy, it is not possible to inhibit the invasion and metastasis of surviving cancer cells even if temporary suppression of cancer is achieved. Thus, gene therapy which is promising for invasion and metastasis of cancer has not been established yet.

In addition, virus vector is mostly used in gene therapy but virus vector is not specific to tumor cells. Therefore, since virus vector having no tumor targeting ability is not able to reach the metastatic diseases, its use is limited to a direct or local administration only.

Gene therapy using DNA coding for NK4 has not been known yet and it is also unknown whether it has a pharmacological action and whether gene therapy is possible.

Further, in the utilization of NK4 as a protein preparation, large amount of NK4 is thought to be necessary. However, it has not been known whether a transformant prepared by transformation of recombinant expression vector where DNA coding for NK4 is integrated in expression vector is able to express the said NK4 having biological activities and whether the NK4 is able to be prepared by using a genetic recombination technique in large amount and in an economical manner.

### SUMMARY OF THE INVENTION

An object of the present invention is to provide a therapeutic agent which is effective for primary tumor of cancer and, more particularly, to provide an NK4 gene therapeutic agent and a recombinant NK4 protein preparation which is effective for prevention and/or therapy of cancer. Another object of the present invention is to provide a therapeutic agent which is effective for metastasis of cancer and, more particularly, to provide an NK4 gene therapeutic agent and a recombinant NK4 protein preparation which is effective for prevention and/or therapy of metastasis of cancer.

Still another object of the present invention is to provide a therapeutic agent which is effective for diseases caused by neovascularization including cancer and, more particularly, to provide an NK4 gene therapeutic agent and a recombinant NK4 protein preparation which is effective for diseases caused by neovascularization.

Further object of the present invention is to provide DNA coding for NK4, recombinant expression vector containing the said DNA, transformant holding the said recombinant expression vector, a process for the production of recombinant NK4 using the said DNA, recombinant NK4 which is produced by the said process for the production, a method for the screening of a substance promoting the binding activity of NK4 with a c-Met/HGF receptor, a substance prepared by the said screening method or a medicament containing the substance prepared by the screening .

The present inventors have carried out intensive investigations for achieving the above objects and found that suppression of tumor growth and metastasis, apoptosis induction and/or suppression of neovascularization are/is achieved by inhibition of invasion and metastasis by the antagonistic activity against HGF and suppression of neovascularization due to a mechanism which is other than the antagonistic activity against HGF when DNA which contains a base sequence represented by SEQ ID NO: 1 or SEQ ID NO: 2 coding for NK4 is introduced into tumor tissue and/or tumor cell, normal tissue and/or normal cell or body.

The present inventors have further found that, when DNA which contains a base sequence represented by SEQ ID NO: 1 or SEQ ID NO: 2 coding for NK4 is integrated in expression vector and transformed to a host, it is now possible to prepare a recombinant NK4 in large amount and in economical manner by making use of genetic recombination technique and that the resulting recombinant NK4 is able to be used for the prevention and therapy of ovary cancer, pancreatic cancer, stomach cancer, gallbladder cancer, breast cancer, colorectal cancer and the like.

The present inventors have carried out further investigations on the basis of such findings and accomplished the present invention.

Thus, the present invention relates to
(1) DNA which contains a base sequence represented by SEQ ID NO: 1 or SEQ ID NO: 2 or DNA which contains DNA hybridizing to the said DNA under a stringent condition;
(2) The DNA according to the above (1), wherein it is DNA which contains a base sequence represented by SEQ ID NO: 1 or SEQ ID NO: 2;
(3) A recombinant expression vector containing the DNA mentioned in the above (1) or (2);
(4) The recombinant expression vector according to the above (3), wherein the expression vector is adeno-associated virus (AAV), retrovirus, poxvirus, herpes virus, herpes simplex virus, lentivirus (HIV), Sendai virus, Epstein-Barr virus (EBV), vaccinia virus, poliovirus, Sindbis virus, SV 40 or plasmid;
(5) Liposome containing the DNA mentioned in the above (1) or (2) or that containing the recombinant expression vector mentioned in the above (3) or (4);
(6) Transformant which is transformed by the recombinant expression vector mentioned in the above (3) or (4);
(7) The transformant according to the above (6), wherein the host is bacterium, yeast, plant cell or mammalian cell;
(8) A process for the production of protein coded to the DNA mentioned in the above (1), characterized in that, the transformant mentioned in the above (6) or (7) is incubated and the protein coded to the DNA mentioned in the above (1) is produced;
(9) Recombinant protein obtained by the process for the production mentioned in the above (8);
(10) Medicament containing the DNA mentioned in the above (1) or (2), the recombinant expression vector mentioned in the above (3) or (4), the liposome mentioned in the above (5), the transformant mentioned in the above (6) or (7) or the recombinant protein mentioned in the above (9);
(11) The medicament according to the above (10), wherein it is an anti-cancer agent;
(12) The medicament according to the above (10), wherein it is an agent for the prevention and/or therapy of ovarian cancer, pancreatic cancer, stomach cancer, gallbladder cancer, renal cancer, prostatic cancer, breast cancer, esophageal cancer, hepatic cancer, oral cavity cancer, colon cancer, colorectal cancer, sarcoma, glioma or melanoma;
(13) The medicament according to the above (10), wherein it is a neovascularization suppressor;
(14) A medicament in which the DNA mentioned in the above (1) or (2), the recombinant expression vector mentioned in the above (3) or (4), the liposome mentioned in the above (5), the transformant mentioned in the above (6) or (7) or the recombinant protein mentioned in the above (9) is combined with other anti-tumor agent;
(15) The medicament according to the above (14), wherein it is an agent for the prevention and/or therapy of ovarian cancer, pancreatic cancer, stomach cancer, gallbladder cancer, renal cancer, prostatic cancer, breast cancer, esophageal cancer, hepatic cancer, oral cavity cancer, colon cancer, colorectal cancer, sarcoma, glioma or melanoma;
(16) A method for the prevention and/or therapy of cancerous diseases, characterized in that, an effective dose of the DNA mentioned in the above (1) or (2), the recombinant expression vector mentioned in the above (3) or (4), the liposome mentioned in claim 5, the transformant mentioned in the above (6) or (7) or the recombinant protein mentioned in the above (9) is administered to mammals;
(17) The method for the prevention and therapy according to the above (16), wherein an effective dose of other anti-tumor agent is further administered;
(18) A method for the prevention of metastasis of cancer, characterized in that, an effective dose of the DNA mentioned in the above (1) or (2), the recombinant expression vector mentioned in the above (3) or (4), the liposome mentioned in the above (5), the transformant mentioned in the above (6) or (7) or the recombinant protein mentioned in the above (9) is administered to mammals;
(19) A method for the suppression of neovascularization, characterized in that, an effective dose of the DNA mentioned in the above (1) or (2), the recombinant expression vector mentioned in the above (3) or (4), the liposome mentioned in the above (5), the transformant mentioned in the above (6) or (7) or the recombinant protein mentioned in the above (9) is administered to mammals;
(20) A method for the screening of a substance which promotes the binding activity of the protein encoded by the DNA mentioned in the above (1) to a c-Met/HGF receptor, characterized in that, the protein encoded by the DNA mentioned in claim 1 and a c-Met/HGF receptor are used;
(21) A substance which promotes the binding activity of the protein encoded by the DNA mentioned in the above (1) to a c-Met/HGF receptor obtained by the method for the screening mentioned in the above (20); and
(22) A medicament containing the substance mentioned in the above (21).

### BRIEF DESCRIPTION OF THE DRAWINGS

Fig. 1 is a drawing which shows the construction schematic of a recombinant adenovirus vector AdCMV.NK4.
Fig. 2 is a drawing which shows the result of tumor invasion suppressive effect of SUIT-2 cells (uninfected cells), SUIT-2 cells infected with AdCMV.LacZ (LacZ 100 MOI) and SUIT-2 cells infected with AdCMV.NK4 (NK4 50 MOI, NK4 100 MOI) in the presence or absence of HGF.
Fig. 3 is a drawing which shows the result of suppressive effect for tumor growth when SUIT-2 cells (a group to which no Ad was infected), SUIT-2 cells infected with AdCMV.LacZ (Ad-lacZ) and SUIT-2 cells infected with AdCMV.NK4 (Ad-NK4) were inoculated to nude mice and then tumor volumes were measured with a lapse of time.
Fig. 4 is a drawing which shows the result of suppressive effect for neovascularization when SUIT-2 cells (a group to which no Ad was infected), SUIT-2 cells infected with AdCMV.LacZ (Ad-lacZ) and SUIT-2 cells infected with AdCMV.NK4 (Ad-NK4) were inoculated to nude mice and then the number of blood vessel was measured after 28 days.
Fig. 5 is a drawing which shows the result of suppressive effect for tumor growth when TMKI cells (P) and TMKI cells (T11) transfected with pcDNA3/NK4 were inoculated to nude mice and tumor volumes were measured with a lapse of time.
Fig. 6 is a drawing which shows the result of produced amount of NK4 after 31 days from the inoculation when TMKI cells (P) and TMKI cells (T11) transfected with pcDNA3/NK4 were inoculated to nude mice.
Fig. 7 is a drawing which shows the result of suppressive effect for tumor growth when TMKI cells were inoculated to nude mice, and then PBS, AdCMV.LacZ (Ad-lacZ) and AdCMV.NK4 (Ad-NK4) were administered after 7 days were elapsed from the inoculation and the tumor volumes were measured with a lapse of time.
Fig. 8 is a drawing which shows the result of produced amount of NK4 after administration of PBS, AdCMV.LacZ (LacZ) and AdCMV.NK4 (NK4) for continuous three days to nude mice to which TMKI cells were inoculated.
Fig. 9 is a drawing which shows a schematic of pCAGGS-DHFR/NK4.
Fig. 10: A is a drawing which shows the result of a suppressive effect of the recombinant NK4 for tumor growth obtained by measuring the tumor volume. B is a drawing which shows a metastasis-suppressing effect of the recombinant NK4 obtained by measuring the number of metastatic nodules.
Fig. 11 is a drawing which shows the life-prolonging effect of the recombinant NK4 when the recombinant NK4 was administered to nude mice of terminal cancer and the number of the survived mice was counted.
Fig. 12 is a drawing which shows the result of suppressive effect for melanoma growth when melanoma cells (a group to which PBS was injected), melanoma cells infected with Ad.CMV.LacZ or melanoma cells infected with Ad.CMV.NK4 were inoculated to mice and then tumor volumes were measured with a lapse of time.
Fig. 13 is a drawing which shows the result of suppressive effect for metastasis when melanoma cells (a group to which PBS was infected), melanoma cells infected with Ad.CMV.LacZ or melanoma cells infected with Ad.CMV.NK4 were inoculated to mice and then the number of lung metastasis were measured after 21 days.
Fig. 14 is a drawing which shows the construction schematic of expression plasmid pCAG-NK4.
Fig. 15 is a drawing which shows the result of suppressive effect for tumor growth when MC-38 cells injected with pCAG-empty plasmid or pCAG-NK4 plasmid were inoculated to mice and then tumor volumes were measured with a lapse of time.
Fig. 16 is a drawing which shows the result of suppressive effect for metastasis when MC-38 cells injected with pCAG-empty plasmid or pCAG-NK4 plasmid Ad.CMV.NK4 were inoculated to mice and then the number of liver metastasis were measured after 21 days.

### DETAILED DESCRIPTION OF THE INVENTION

The base sequence represented by SEQ ID NO: 1 is an example of DNA coding for NK4. NK4 is a protein comprising the N-terminal hairpin domain and subsequent four-Kringle domains of α-chain of HGF. NK4 binds to the c-Met/HGF receptor and suppresses invasion and metastasis of tumor by an HGF-antagonist activity. It also shows a neovascularization suppressive action due to a mechanism other than the HGF-antagonist activity.

A base sequence represented by SEQ ID NO: 2 is an example of DNA coding for NK4. In the base sequence represented by SEQ ID NO: 2, the 391st to the 405th bases in the base sequence represented by SEQ ID NO: 1 are deficient. Protein which is produced by DNA containing a base sequence represented by SEQ ID NO: 2 also has an HGF-antagonist activity and neovascularization suppressive action.

Besides the DNA having a base sequence represented by the above SEQ ID NO: 1 or SEQ ID NO: 2 in the present invention, a DNA which hybridizes to the above-mentioned DNA under a stringent condition is also within a scope of the DNA coding for NK4 in accordance with the present invention. Thus, even if the DNA sequence is partially changed by various artificial treatments such as introduction of site-specific mutation, random mutation by treating with mutagen and mutation, deletion, connection, etc. of DNA fragments due to cleavage by restriction enzyme, that is still within a scope of the DNA coding for NK4 in accordance with the present invention so far as such a DNA mutant hybridizes under a stringent condition to DNA containing a base sequence represented by SEQ ID NO: 1 or SEQ ID NO: 2 and is able to produce a protein having an HGF-antagonist activity and a neovascularization suppressive action.

To be more specific, since there are generally plural kinds of codons for one amino acid, even a DNA containing a base sequence which is different from that of the DNA containing a base sequence represented by SEQ ID NO: 1 or SEQ ID NO: 2, it is still within a scope of the DNA coding for NK4 in accordance with the present invention so far as it is able to produce a protein having an HGF-antagonist activity and a neovascularization suppressive action.

The DNA which hybridizes under a stringent condition means a DNA which is prepared by colony hybridization method, plaque hybridization method, southern blot hybridization and the like using the above-mentioned DNA as a probe.

To be more specific, there may be exemplified a DNA which can be identified in such a manner that hybridization is carried out at about 65°C in the presence of about 0.7 to 1.0 M sodium chloride using a filter where DNA derived from colonies or plaques is fixed and then the filter is washed under the condition of about 65°C using an SSC solution having about 0.1- to 2-fold concentration (composition of the SSC solution of 1-fold concentration comprises 150 mM sodium chloride and 15 mM sodium citrate). Hybridization may be carried out by a method according to a method described in "Molecular Cloning", second edition, Current Protocols in Molecular Biology, DNA Cloning I: Core Techniques, A Practical Approach, second edition, Oxford University (1995), etc.

With regard to the above-mentioned DNA which hybridizes to a base sequence represented by SEQ ID NO: 1 or SEQ ID NO: 2, there may be specifically exemplified DNA having at least not less than about 70% of homology, preferably not less than about 80% of homology, more preferably not less than about 90% of homology or, most preferably, not less than about 95% of homology to the base sequence of DNA having a base sequence represented by SEQ ID NO: 1 or SEQ ID NO: 2.

Further, protein which is coded by DNA hybridizing under a stringent condition to DNA containing a base sequence represented by the SEQ ID NO: 1 or SEQ ID NO: 2 and has an HGF-antagonist activity and a neovascularization suppressive action is also within a scope of the NK4 of the present invention.

Thus, even in the case of a protein which has an amino acid sequence where one or more amino acid(s) is/are deleted, substituted and/or added in the amino acid sequence of NK4, such a protein is still within a scope of NK4 of the present invention so far as it has an HGF-antagonist activity and a neovascularization suppressive action.

Such a protein is able to be manufactured by introduction of a site-specific mutation to DNA coding for the above-mentioned protein having an HGF-antagonist activity and a neovascularization suppressive activity using a method for the introduction of a site-specific mutation mentioned, for example, in Molecular Cloning, A Laboratory Manual, Second Edition, Cold Spring Harbor Laboratory Press (1989); Current Products in Molecular Biology, John Wiley & Sons (1987-1997); *Nucleic Acids Research,* **10**, 6487 (1982) ; *Proc. Natl. Acad. Sci., USA,* **79**, 6409(1982) ; *Gene,* **34**, 315(1985) ; *Nucleic Acids Research*, **13**, 4431(1985); and *Proc. Natl. Acad. Sci., USA,* **82**, 488(1985).

Although there is no particular limitation for the number(s) of the amino acid(s) which is/are deleted, substituted and/or added, it is preferred to be one to several dozen, preferably from 1 to 30, more preferably from 1 to 10 and, particularly preferably, from 1 to several amino acid(s). Although there is no particular limitation for the number(s) of the base (s) which is/are deleted, substituted and/or added, it is preferred to be one to several dozen, preferably from 1 to 90, more preferably from 1 to 30 and, particularly preferably, from 1 to several amino acid(s).

A protein having a modified amino acid residue pyroglutamate as an N-terminal amino acid is also within a scope of the NK4 of the present invention.

Incidentally, the following fundamental operations for genetic engineering or biotechnology may be carried out according to a method mentioned in commercially available books on experiments such as Manual for Gene, Kodansha; Method for Experiments for Genetic Operation, edited by Yasutaka Takagi, Kodansha; Molecular Cloning, Cold Spring Harbor Laboratory (1982); Molecular Cloning, second edition, Cold Spring Harbor Laboratory (1989); *Methods in Enzymology,* **194,** (1991); and Supplementary Issue of *Jikken Igaku,* Method for Gene Experiments by Yeast, Yodosha (1994).

It is at first necessary to prepare DNA coding for the above-mentioned NK4. DNA coding for NK4 may, for example, be prepared as follows.

Thus, for example, a primer is prepared on the basis of a known base sequence for HGF and then amplification of DNA is carried out using a PCR (PCR Protocols, Academic Press (1990)) using cDNA which is synthesized from HGF mRNA contained in human tissues or cells or using an HGF cDNA selected from a cDNA library as a template whereupon the above mentioned DNA coding for NK4 can be obtained. Vector used for the library may be any of bacteriophage, plasmid, cosmid, phagemid and the like. It is also possible to directly amplify by an RT-PCR from mRNA contained in human tissues or cells. It is further possible to prepare by means of chemical synthesis using an already-known method from the known base sequence information of HGF. With regard to a chemical synthetic method, there may be exemplified a method where the chemical synthesis is carried out using a DNA synthesizer such as DNA Synthesizer Model 392 (manufactured by Perkin Elmer) utilizing a phosphoamidite method.

Other examples are a method where an aimed DNA is amplified from a cDNA library by a PCR using a synthetic DNA primer having a partial base sequence of DNA coding for known HGF amino acid sequence, or a method where screening of an aimed DNA is carried out by hybridization to labeled DNA fragments of HGF or synthetic DNA (probe).

After that, there is constructed a recombinant expression vector containing DNA coding for NK4 which was prepared as mentioned above. The recombinant expression vector of the present invention is constituted from DNA coding for the above NK4 and an expression vector for the expression of the said DNA.

DNA coding for NK4 can be prepared by the above-mentioned method.

DNA coding for NK4 maybe used either as it is or, if desired, after digesting with a restriction enzyme or after adding a linker. The said DNA may have ATG as a translation initiation codon at the 5'-terminal side and may also have TAA, TAG or TGA as a translation termination codon at the 3'-terminal side. Such translation initiation codon and translation termination codon may be added to the said DNA by using an appropriate DNA adaptor. It is preferred that a polyadenylated sequence is available at the 5'-terminal side.

In the expression vector, a regulatory sequence is usually added to an expression vector in order to express DNA coding for NK4 or to make it advantageous for the expression. Each regulatory sequence may be intrinsic or extrinsic to the vector .

With regard to such a regulatory sequence, there may be exemplified, although not limitative, signal sequence, promoter, propeptide sequence, enhancer, selective marker and terminator. Regulatory sequence may have a linker whereby the linkage to DNA coding for NK4 and the linkage between the above-mentioned regulator sequences become easy.

In the meanwhile, when a signal sequence is integrated, NK4 produced in organism cells or host cells can be secreted outside the organism cells or host cells. In other words, by a signal sequence, NK4 is produced in a form of being added with a signal peptide and, as a result, NK4 is able to be secreted outside the organism cells or host cells. NK4 is a secretory protein and a signal sequence is essential for secreting the NK4 outside the organism. NK4 is positively secreted outside the cells by the said signal sequence and it is now possible by NK4 to conduct suppression of tumor invasion, growth and metastasis, apoptosis induction and/or suppression of neovascularization even to the far distant cells other than the said cells.

With regard to a method for adding a signal sequence, it is possible to use a method which is known *per se* and there may be exemplified the methods mentioned in *J. Biol. Chem.,* **264,** 17619 (1989); *Proc. Natl. Acad. Sci., USA*, **86**, 8227 (1989) ; *Genes Develop.,* **4**, 1288 (1990); Japanese Patent Laid-Open No. 05/336963; WO 94/23021; etc.

The signal sequence is preferably recognized by host cells and is subjected to processing. When the host is *Escherichia,* there maybe utilized PhoA signal sequence, OmpA signal sequence, etc; when the host is *Bacillus,* there may be utilized α-amylase signal sequence, subtilisin signal sequence, etc.; when the host is yeast, there may be used MFα signal sequence, SUC2 signal sequence, etc.; and, when the host is mammal cells, there may be used HGF signal sequence, insulin signal sequence, α-interferon signal sequence, etc. In the present invention, utilization of HGF signal sequence derived from human being is particularly preferred.

Promoter is an untranslated sequence locating at the upper stream of translation initiation codon and controls the transcription of specific DNA.

With regard to the promoter used in the present invention, anything may be used so far as it is a promoter which is appropriate being corresponding to the host used for expression of gene. For example, when the host is *Escherichia*, the preferred ones are trp promoter, lac promoter, rec A promoter, λP_{L} promoter, lpp promoter, etc.; when the host is *Bacillus,* the preferred ones are SPO1 promoter, SPO2 promoter, penP promoter, etc; and, when the host is yeast, the preferred ones are PHO5 promoter, PGK promoter, GAP promoter, ADH promoter, etc.

When mammalian cells are used as a host, there may be exemplified promoters obtained from virus genome such as Rouse sarcoma virus (virus RSV), MPSV, polyoma virus, fowl pox virus, adenovirus, bovine papilloma virus, avian sarcoma virus, cytomegalovirus (CMV), hepatitis B virus, simian virus 40 (SV 40), vaccinia virus and the like, merothioneine promoter, heat shock promoter, etc. When higher mammalian host is used, it is preferred that enhancer is introduced into vector. As a result of introduction of the enhancer, transcription increases. Examples of the enhancer are SV40 enhancer, initial promoter/enhancer of cytomegalovirus, polyoma enhancer, enhancer of adenovirus, etc.

With regard to a selective marker, there maybe exemplified dihydrofolic acid reductase gene (resisting to methotrexate (MTX)), ampicillin-resistant gene and neomycin-resistant gene. Especially when DHFR gene is used as a selective marker using CHO (DHFR⁻) cells, the aimed gene can be selected even by using a medium containing no thymidine.

In general, recombinant expression vector can be constructed using DNA or RNA virus vector or plasmid vector which is able to express protein in bacteria, yeasts, filamentous fungi, plant cells, mammalian cells, etc. Recombinant expression vector can be constructed, for example, by linking of DNA coding for aimed NK4 with an appropriate expression vector. Recombinant expression vector can be constructed in accordance with a known technique (Molecular Cloning, Cold Spring Harbor Laboratory, A Laboratory Manual (1989)).

With regard to an expression vector, there may be used plasmid derived from *Escherichia coli* such as pCR4, pCR2.1, pBR 322, pBR325, pUC12 and pUC13; plasmid derived from *Bacillus subtilis* such as pUB110, pTP5 and pC194; plasmid derived from yeast such as pSH19 and pSH15; bacteriophage such as λ phage; animal virus such as retrovirus and vaccinia virus; and others such as pA1-11, pXT1, pRc/CMV, pRc/RSV and pcDNAI/Neo.

The DNA coding for the above NK4, recombinant expression vector containing the said DNA or artificial vector (such as liposome) containing the said DNA is safe and has low toxicity and, therefore, it can be administered to, for example, mammals (such as human being, rats, mice, rabbits, sheep, swine, cattle, cats, dogs and monkeys and the like).

When it is used for gene therapy, it is preferred to use DNA or RNA virus vector or plasmid vector which is able to express protein in cells of mammals including human being and has high safety.

Examples of the virus vector which is preferred in gene therapy are adenovirus, adeno-associated virus (AAV), retrovirus, pox virus, herpes virus, herpes simplex virus, lentivirus (HIV), Sendai virus, Epstein-Barr virus (EBV), vaccinia virus, poliovirus, sindbis virus and SV 40.

More preferred examples are adeno-associated virus or adenovirus. There are various serum types in adenovirus and, in the present invention, it is preferred to use human adenovirus of type 2 or type 5. It has been known that adenovirus has higher infecting efficiency as compared with other virus vectors, that it is able to infect non-dividing cells and that it is not recombined in genome of cells and, from the above point of view, it is more preferred to use adenovirus vector.

With regard to a virus vector, replicative defective virus where virus gene is completely or almost completely deficient is preferred. It is preferred that at least E1 region of adenovirus vector is non-functional. Other regions may be modified as well and, particularly, any of E3 region (WO 95/02697), E2 region (WO 94/28938), E4 region (WO 94/28152, WO 94/12649 and WO 95/02697) and late gene L1 to L5 may be modified. Modified virus vector such as replicative defective virus may be prepared by a method which has been known per se. In addition, modified virus vector may be recovered and purified by a method which has been known *per se.* It is also possible to use the modified virus vector mentioned, for example, in Japanese Patent Laid-OpenNos. 11/514,866, 11/506,311, 09/500,524, 08/501,703, 08/508,648 and 08/308,575.

When DNA coding for NK4 is introduced into such a modified virus vector and infected to organism or cells, it is possible to introduce gene into organism or cells.

Methods for the preparation of virus vector and for the introduction of gene are mentioned in Supplementary Issue of *Jikken Igaku,* Fundamental Technique of Gene Therapy, Yodosha (1996) or Supplementary Issue of *Jikken Igaku,* Method of Analytical Experiment of Introduction and Expression of Gene, Yodosha (1997), etc.

It is also possible that expression vector is introduced *in vivo* as a bare plasmid. Examples of preferred plasmid in gene therapy are pCAGGS *(Gene,* **108,** 193-200 (1991)), pBK-CMV, pcDNA3.1, pZeoSV (Invitrogen, Stragene), Japanese Patent Laid-Open No. 11/511,009, etc. Bare plasmid for gene therapy can be introduced into cells by a known method *per se* such as transfection, electroporation, microinjection, transduction, cell fusion, DEAE dextran, calcium phosphate precipitation, a method for the introduction of DNA into cells together with a carrier (metal particles) by gene gun, etc. (Wu et al., *J. Biol. Chem*., **267**, 963-967 (1992) ; Wu et al., *J. Biol. Chem*., **263**, 14621-14624, (1988); *Proc. Natl. Acad. Sci., USA,* **88**, 2726-2730 (1991)).

Examples of the artificial vector are liposome, microcapsule, cytofectin, DNA-protein complex and biopolymer.

Liposome is a closed endoplasmic reticulum made of double membrane of lipid having an aqueous layer inside and its lipid bimolecular membrane structure is known to be quite similar to biomembrane. Examples of phospholipid used for the manufacture of liposome are phosphatidylcholine such as lecithin and lysolecithin; acidic phospholipid such as phosphatidylserine and phosphatidylglycerol; and sphingophospholipid such as phosphatidylethanolamine and sphingomyelin. It is also possible to add cholesterol or the like. Liposome can be manufactured by a known method *per se.* With regard to liposome, there has been known membrane-fused liposome, HVJ-membrane fused liposome (Kaneda, Y., et al., *Biol. Chem.,* **264,** 12126-12129(1989); Kato, K., et al., *Biol. Chem.,* **266,** 3361-3364(1991); Tomita, N., et al., *Biochem. Biophys. Res.,* **186,** 129-134, (1992); Tomota, N., et al., *Cric. Res.,* **73,** 898-905(1993); cationic liposome (Japanese Patent Laid-Open Nos. 2000/510,151 and 2000/516,630), etc. It is particularly preferred to use HVJ-membrane fused liposome in which liposome is fused with Sendai virus (HVJ). When surface of liposome is integrated with or covalently bonded to glycoprotein of HVJ and polyethylene glycol or the like is added to it, then efficiency of introduction of gene into cells is improved.

When signal sequence, promoter and polyadenylated sequence are added to DNA coding for NK4 and the resulting DNA is contained in liposome, then an agent for gene therapy according to the present invention can be prepared. It is also possible to prepare an agent for gene therapy according to the present invention when a recombinant expression vector is contained in liposome.

With regard to a method for the introduction of DNA using liposome, there may be exemplified liposome method, HVJ-liposome method, cationic liposome method, lipofectin method and lipofectamine method.

Microcapsule is a film-coated particle and consists of particle, etc. coated with a coating material comprising mixture of membrane-forming polymer derivative, hydrophobic plasticizer, surface-active agent and/or lubricant nitrogen-containing polymer. Japanese Patent Laid-Open No. 2000/500,744, etc. are advantageously used for gene therapy.

When signal sequence, promoter and polyadenylated sequence are added to DNA coding for NK4 and the resulting DNA is contained in microcapsule, an agent for gene therapy according to the present invention can be prepared. It is also possible to prepare an agent for gene therapy according to the present invention when a recombinant expression vector is contained in microcapsule.

A transformant where a recombinant expression vector containing DNA coding for NK4 is introduced into host cells can be used as agent for gene therapy of the present invention as well. With regard to the host, there may be used bifidbacterium, lactobacillus, yeasts, filamentous fungi and the like. Preferably used ones are bifidbacterium and lactobacillus. With regard to the bifidbacteria, there may be exemplified *Bifidobacterium longum, Bifidobacterium bifidum, Bifidobacterium breve,* etc. With regard to the lactobacillus, there may be exemplified *Lactobacillus, Streptococcus, Leuconostoc, Pediococcus,* etc. It is also possible that the transformant is contained in a capsule to give an agent for gene therapy of the present invention.

With regard to the form of the capsule, three-layered structure is preferably used. In the case of a three-layered structure, a transformant is an innermost layer, an intermediate layer coating the said transformant is a lipophilic membrane and the outermost layer is selected, if necessary, from outer film which is soluble in the mouth, outer film which is soluble in stomach, outer film which is soluble in small intestine, outer film which is soluble in large intestine, etc. The innermost layer is a transformant together, if necessary, with water, physiological saline, buffer, culture medium, etc. With regard to the lipophilic membrane of the intermediate layer, there may be used animal fat/oil, vegetable fat/oil, biologically or chemically treated fat/oil thereof, etc. With regard to the outer membrane, it is preferred to use natural high-molecular coat such as gelatin, agar, pectin and alginic acid or high-molecular coat composed of the substances obtained, if necessary, by adding protein, glycoprotein, mucopolysaccharide, sugar, sugar alcohol, polyhydric alcohol, etc. to natural one. Such a capsule may be manufactured according to a known method.

With regard to a method for the administration of a gene therapy agent of the present invention to a patient, there are an *in vivo* method where the gene therapy agent is directly introduced into the body and an *ex vivo* method where a predetermined cell is taken out from human body, DNA is introduced into the said cell in vitro and the said cell is inoculated back to the body (*Nikkei Science,* April issue, 20-45 (1994); *Gekkan Yakuji,* **36,** 23-48 (1994) ; and extra issue of *Jikken Igaku,* **12,** 15 (1994)). In the present invention, an *in vivo* method is preferred.

When administration is carried out by an *in vivo* method, the administration is done by an appropriate administration route depending upon the disease to be treated, target organ, etc. For example it is possible that a direct intratumoral administration or topical administration is conducted to the diseased tissues or that administration is conducted by, for example, way of intravenous, intra-arterial, subcutaneous, intramuscular, intraperitoneal, endoscopic or aerosolic means. With regard to the administration method, intravenous or intraperitoneal administration is preferred. A direct injection to diseased tissues, particularly to tumor itself, is preferred as well. It is also possible to administer the gene therapy agent of the present invention in such a manner that anything which is able to be utilized in the said technical field such as nuclear magnetic resonance imaging or computed tomography is used to take the image of tumor followed by, for example, conducting a stereotaxic injection.

In the meanwhile, administration into tumor tissue or tumor cell or topical administration is not always necessary when NK4 is altered into secretory protein by adding signal sequence to DNA coding for NK4. Because secretory protein produced and secreted in the cells acts on a target organ which is far distantly located resulting in the action of suppression of tumor invasion, growth and metastasis, apoptosis induction and/or suppression of neovascularization. Accordingly, it is now possible to administer into normal tissue or normal cell other than tumor tissue. Incidentally, in the case of administering to human being, intravenous administration or intramuscular administration is preferred.

With regard to a preparation form, it is possible to adopt various preparation forms corresponding to each of the above-mentioned administration mode. For example, in the case of parenteral injection containing DNA of the present invention which is an effective ingredient, the said parenteral injection may be prepared by conventional methods. With regard to a base material used for a gene therapy agent, there is no particular limitation so far as a base material is commonly used for parenteral injection and its examples are distilled water, salt solution of sodium chloride or of a mixture of sodium chloride with inorganic acid, etc., solution of mannitol, lactose, dextran, glucose, etc., solution of amino acid such as glycine and arginine, mixed solution of organic acid solution or salt solution with a glucose solution. It is also possible that, according to conventional methods, adjuvant such as osmotic pressure adjusting agent, pH adjusting agent, vegetable oil such as sesame oil and soybean oil or lecithin or surfactant such as nonionic surfactant etc. is added to such a base material to prepare solution, suspension or dispersion whereby a parenteral injection is prepared. Such a parenteral injection may be pulverized, freeze-dried, etc. to give a preparation which is dissolved in actual use.

In the case of a liposome preparation in such forms as HVJ-liposome, it is possible to be a liposome preparation in such forms as suspension, frozen agent and centrifugally concentrated and frozen agent.

In addition, in the case of microcapsule preparation, it is possible that a preparation of a sustained release type, for example, is prepared and is made into an oral formulation or inoculated into the diseased part, under the skin, into the muscle, etc. whereby the gene is made to be apt to exist around the diseased part.

Amount of DNA in the preparation may vary depending upon the disease to be treated, site to be administered, administering frequency, desired therapeutic period, age and body weight of a patient, etc. and may be appropriately adjusted. Usually, however, in the case of patients suffering from cancer (body weight: 60 kg), dosage of DNA coding for NK4 is generally about 0.01 to 2,000 mg or, preferably, 0.1 to 100 mg. In the case of other animals, it is also possible to administer the dosage which is calculated on the basis of the above mentioned dosage for body weight of 60 kg.

The NK4 gene therapeutic agent of the present invention as mentioned above is able to be used as a preventive and therapeutic agent for the diseases caused by cancer and/or neovascularization and as a preventive agent for metastasis of cancer. Accordingly, the NK4 gene therapeutic agent of the present invention is able to be used for the suppression of tumor invasion, growth and metastasis, apoptosis induction and/or suppression of neovascularization. In addition, the NK4 gene therapeutic agent of the present invention is able to provide a method for the suppression of tumor invasion, growth and metastasis, apoptosis induction and/or suppression of neovascularization.

With regard to the cancer which is an object disease, there are exemplified lung cancer, ovarian cancer, pancreatic cancer, stomach cancer, gallbladder cancer, renal cancer, prostatic cancer, breast cancer, esophageal cancer, hepatic cancer, oral cavity cancer, colon cancer, colorectal cancer, uterine cancer, cholangioma, insulinoma, adrenocortical cancer, bladder cancer, testicular cancer, testicular tumor, thyroid cancer, skin cancer, malignant carcinoid tumor, malignant melanoma, osteosarcoma, soft tissue tumor, neuroblastoma, Wilms tumor, retinoblastoma, melanoma and glioma. Among them, ovarian cancer, pancreatic cancer, stomach cancer, gallbladder cancer, renal cancer, prostatic cancer, breast cancer, esophageal cancer, hepatic cancer, oral cavity cancer, colon cancer, colorectal cancer, sarcoma, melanoma or glioma is preferred and particularly preferred ones are ovarian cancer, pancreatic cancer, stomach cancer and gallbladder cancer.

With regard to the disease caused by neovascularization, there are exemplified rheumatic arthritis, psoriasis, Osler-Webber syndrome, angiogenesis of heart muscle, peripheral hemangiectasis, hemophilic arthritis, angiogenesis of eye (such as diabetic retinopathy, retinopathy of prematurity, age-related macular degeneration, rejection to keratoplasty, neovascular glaucoma, retrolental fibroplasia and perosis), angiofibroma, benign tumor (such as hemangioma, acoustic neuroma, neurofibroma, trachoma and suppurative granuloma), tumor of hematopoietic organs including leukemia, solid tumor, metastasis of tumor and granulation after wound etc.

It is also possible that the NK4 gene therapeutic agent of the present invention is used in combination with other anti-tumor agent and the like. It is generally preferred to use in combination with several kinds of anti-tumor agents having different action mechanisms.

Examples of other anti-tumor agent as such are alkylating agents, various kinds of antimetabolites, anti-tumor antibiotic substances and other anti-tumor agents, anti-tumor plant components, BRM (biological response modifiers), cell adhesion inhibitors, matrix metalloprotease inhibitors, hormones, vitamins, antibacterial antibiotic substances and chemotherapeutic agents.

To be more specific, examples of the alkylating agents are alkylating agent such as nitrogen mustard, nitrogen mustard N-oxide and chlorambucil; alkylating agent of aziridine type such as carboquone and thiotepa; alkylating agent of epoxide type such as dibromomannitol and dibromodulcitol; alkylating agent of nitrosourea type such as carmustine, lomustine, semustine, nimustine hydrochloride, streptozocin, chlorozotocin and ranimustine; busulfan; improsulfan tosylate; dacarbazine; etc.

Examples of various kinds of antimetabolites are antagonist for purine metabolism such as 6-mercaptopurine, 6-thioguanine and thioinosine; antagonist for pyrimidine metabolism such as fluorouracil, tegafur, tegafur-uracil, carmofur, doxifluridine, broxuridine, cytarabine and enocitabine; antagonist for folic acid metabolism such as methotrexate and trimetrexate; and salt and complex thereof.

Examples of the anti-tumor antibiotic substances are antibiotic anti-tumor agent of anthracycline type such as mitomycin C, bleomycin, peplomycin, daunorubicin, aclarubicin, doxorubicin, pirarubicin, THP-adriamycin, 4'-epidoxorubicin and epirubicin; chromomycin A₃; actinomycin D; and salt of complex thereof.

Examples of other anti-tumor agents are cisplatin, carboplatin, tamoxifen, camptothecin, iphosphamide, cyclophosphamide, melfalan, L-asparaginase, acecraton, schizophyllan, Picibanil, ubenimex, Krestin and salt and complex thereof. Other examples are procarbazine, pipobroman, neocarzinostatin and hydroxyurea.

Examples of the anti-tumor vegetable components are Vinca alkaloid such as vindesine, vincristine and vinblastine; epipodophyllotoxin such as etoposide and teniposide; and salt and complex thereof.

Examples of the BRM are tumor necrosis factor, indomethacin and salt or complex thereof.

Examples of the cell adhesion inhibitors are a substance having an RGD sequence and salt or complex thereof.

Examples of the matrix metalloprotease inhibitors are marimastat, batimastat and salt or complex thereof.

Examples of the hormones are hydrocortisone, dexamethasone, methylprednisolone, prednisolone, prasterone, betamethasone, triamcinolone, oxymetholone, nandrolone, metenolone, phosphestrol, ethynylestradiol, chlormadinone, medroxyprogesterone and salt or complex thereof.

Examples of the vitamins are vitamin C, vitamin A and salt or complex thereof.

Further, the NK4 gene therapy agent of the present invention may also be used in combination with surgical therapy or with radiotherapy. Examples of the radiotherapy are gamma-ray irradiation, X-ray, UV irradiation, microwave and electronic ray irradiation.

Accordingly, the NK4 gene therapy agent of the present invention is able to be used as a preventive and/or therapeutic agent for diseases caused by cancer and/or neovascularization in combination with other anti-tumor agent, etc. and/or surgical therapy or radiotherapy and also as a preventive agent for metastasis of cancer. Consequently, the NK4 gene therapy agent of the present invention is able to be used for the suppression of tumor invasion, growth and metastasis, apoptosis induction and/or suppression of neovascularization in combination with other anti-tumor agent, etc. and/or surgical therapy or radiotherapy. Moreover, the NK4 gene therapy of the present invention is able to provide a method for the suppression of tumor invasion, growth and metastasis, apoptosis induction and/or suppression of neovascularization in combination with other anti-tumor agent, etc. and/or surgical therapy or radiotherapy.

It is possible in the present invention to obtain a recombinant NK4 in large amount and in economical manner by utilization of a gene recombination technique when a recombinant expression vector containing DNA coding for NK4 is transformed to a host.

The recombinant expression vector containing the DNA coding for NK4 is introduced into host cells whereupon a transformant is constructed.

With regard to a method for the introduction of the recombinant expression vector into the host, any method may be used so far as it is a known method *per se.* There may be exemplified a competent cell method (*J. Mol. Biol*., **53,** 154 (1970)), a DEAE dextran method *(Science,* **215,** 166 (1982)), an *in vitro* packaging method (*Proc. Natl. Acad. Sci., USA,* **72**, 581 (1975)), a virus vector method (*Cell,* **37,** 1053 (1984)), a microinjection method (*Exp. Cell. Res.,* **153,** 347 (1984)), an electroporation method (*Cytotechnology,* **3**, 133 (1990)), a calcium phosphate method (*Science,* **221,** 551 (1983)), a lipofection method (*Proc. Natl. Acad. Sci., USA,* **84**, 7413 (1987)), a protoplast method (Japanese Patent Laid-Open No. 63/248394) and a method mentioned in *Gene,* **17,** 107 (1982) and *Molecular & General Genetics*, **168**, 111 (1979).

Examples of the host are bacteria, yeasts, filamentous fungi, plant cells and mammalian cells. Examples of the bacteria are *Escherichia, Enterobacter, Proteus, Salmonella, Serratia, Bacillus, Lactobacillus, Bifidobacterium, Pseudomonas, Streptomyces, Streptococcus, Leuconostoc* and *Pediococcus.*

Examples of the yeasts are *Saccharomyces cerevisiae, Schizosaccharomyces pombe,* NCYC 1913, NCYC 2036, *Pichia pastoris* and bakery yeast.

Examples of the filamentous fungi are *Aspergillus* and *Penicillium.*

Examples of the plant cells are cotton, corn, potato, broad bean, petunia, tomato and tobacco.

Examples of the mammalian cells are mouse C127 cells, Chinese hamster CHO cells, simian COS cells, mouse cells BALB/3T3, mouse L cells, mouse AtT-20, mouse myeloma cells, rat GH3, human HeLa cells, human FL cells and 293 cells derived from kidney of human embryo (*Jikken Igaku,* **12,** 316 (1994)).

The resulting transformant is incubated in an appropriate medium corresponding to the host for the production of the recombinant NK4. The medium contains carbon source, inorganic substances, vitamins, serum, pharmaceuticals, etc. which are necessary for the growth of the said transformant.

When the host for the transformation is *Escherichia coli*, there may be used an LB medium (Nissui Seiyaku), an M9 medium (*J. Exp. Mol. Genet.,* Cold Spring Laboratory, New York, 431 (1972)), etc.; when the host is yeast, there may be used a YEPD medium (Genetic Engineering, vol. 1, Plenum Press, New York, 117 (1979)), etc.; and when the host is animal cell, there may be used an MEM medium containing 20% or less fetal bovine serum, a DMEM medium, a PRMI 1640 medium (Nissui Seiyaku), etc. although the medium is not limited thereto. Incubation of the transformant is usually carried out at 20°C to 45°C with a pH range of 5 to 8 and, if necessary, aeration and stirring are carried out although incubation method is not limited thereto. When the host is adhesive animal cells etc., a support such as glass beads, collagen beads or acetylcellulose hollow fiber may be used if necessary.

The transformant which produces the recombinant NK4 secretes the recombinant NK4 into a supernatant of its culture medium and, therefore, it is possible to extract the recombinant NK4 using the supernatant liquid of the culture medium of the transformant. It is also possible to extract the recombinant NK4 produced in the transformant. In extracting the protein from the incubated bacteria or cells, there is appropriately used, for example, a method where bacteria or cells are collected after the incubation by a known method, suspended in an appropriate buffer, disrupted by sonication, lysozyme and/or freeze-thawing, etc. and centrifuged or filtered to provide a crude extract of the recombinant NK4. Protein-denaturing agent such as urea or guanidine hydrochloride and surfactant such as Triton X-100™ may be contained in the buffer. Purification of the recombinant NK4 contained in the supernatant liquid of the medium or the extract prepared as such may be carried out by an appropriate combination of separation and purifying methods which has been known *per se*. Examples of such known separation and purification methods are a method where difference in solubility is utilized such as salting-out and solvent precipitation method; a method where difference in molecular weights is mostly utilized such as dialysis, ultrafiltration, gel filtration and SDS-polyacrylamide gel electrophoresis; a method where difference in the charge is utilized such as ion-exchange chromatography; a method where specific affinity is utilized such as affinity chromatography; a method where difference in hydrophobicity is utilized such as reversed phase high performance liquid chromatography; and a method where difference in isoelectric point is utilized such as isoelectric electrophoresis.

The recombinant NK4 which is obtained as such is within a scope of the present invention even when it is in a form of salt.

The recombinant NK4 produced by the present invention is safe and has a low toxicity and, therefore, it is able to be administered, for example, to mammals (e.g., human being, rats, mice, rabbits, sheep, swine, cattle, cats, dogs, monkeys and etc.).

The anti-cancer agent or a neovascularization suppressor containing the recombinant NK4 may be in various preparation forms such as liquid, solid and capsule and, generally, only the recombinant NK4 which is an effective ingredient or that with a conventionally used carrier is made into a parenteral injection or that with a conventionally used carrier is made into an oral preparation or a sustained released preparation. The said parenteral injection may be prepared by a conventional method and, for example, it may be prepared in such a manner that the recombinant NK4 is dissolved in an appropriate solvent such as sterilized water, buffer, physiological saline, etc., sterilized by filtration through a filter or the like, and charged into a sterilized container. With regard to the amount of the recombinant NK4 in the parenteral injection, it is usually made about 0.01 to 5.0 (w/v%) in the case of a cancer patient (body weight being 60 kg). As to an oral preparation, it is made into a preparation form such as, for example, tablets, granules, fine granules, diluted powder, soft or hard capsules, liquid, emulsion, suspension or syrup. As to a sustained-released agent, it is made into a preparation form such as, for example, tablets, granules, fine granules, diluted powder, soft or hard capsules or microcapsules. Such a preparation may be prepared according to the conventional method for the manufacture of pharmaceutical preparations. Amount of the recombinant NK4 in the preparation may be appropriately adjusted depending upon the preparation form, diseases to which the preparation is applied, etc.

In preparing the preparation, it is preferred to add a stabilizer and examples of the stabilizer are albumin, globulin, gelatin, mannitol, glucose, dextran and ethylene glycol. It is also possible that the preparation of the present invention may be compounded with an additive which is necessary for preparing the preparation such as filler, solubilizing aid, antioxidant, anesthetizing agent and isotonizing agent. When a liquid preparation is prepared, it is preferred to preserve after removal of water by, for example, means of cryopreservation or freeze-drying etc. A freeze-drying agent is used by re-dissolving, for example, in distilled water for injection upon actual use. When a sustained released preparation is prepared, there may be used a carrier for the sustained release such as soluble collagen or soluble collagen derivative, protein such as gelatin, porous ceramics, polyamino acid, polylactic acid, chitin or chitin derivative and water-swelling high-molecular substance.

The preparation of the present invention may be administered by an appropriate administration route depending upon the form of the said preparation. For example, it may be administered intravenously, intra-arterially, subcutaneously, intramuscularly, etc. after making into a form of parenteral injection or it may be administered by inoculating into body, for example, into diseased part, under the skin, into muscle, etc. after making into a form of a sustained released preparation. Dosage may be appropriately adjusted depending upon symptom, age, body weight, etc. of the patient and, in the case of a cancer patient (body weight being 60 kg), it is usually about 1 mg to 300 mg or preferably about 10 mg to 100 mg as the amount of the recombinant NK4 and it is appropriate that the dosage is administered once or by dividing into several times a day. In the case of other animals, the dosage calculated in terms of per 60 kg body weight may be administered as well.

The recombinant NK4 protein preparation of the present invention is able to be used as a preventive/therapeutic agent for diseases caused by cancer and/or neovascularization and as a preventive agent for metastasis of cancer. Accordingly, the recombinant NK4 protein preparation of the present invention is able to be used for the suppression of tumor invasion, growth and metastasis, apoptosis induction and/or suppression of neovascularization. Further, the recombinant NK4 protein preparation of the present invention is able to provide a method for the suppression of tumor invasion, growth and metastasis, apoptosis induction and/or suppression of neovascularization. Examples of the disease caused by cancer and neovascularization are the above-mentioned diseases.

Furthermore, the recombinant NK4 protein preparation of the present invention may be used in combination with other anti-tumor agent, etc. Examples of other anti-tumor agent are the above-mentioned anti-tumor agents, etc.

Still further, the recombinant NK4 protein preparation of the present invention may be used in combination with surgical therapy or radiotherapy.

Accordingly, the recombinant NK4 protein preparation of the present invention is able to be used for the suppression of tumor invasion, growth and metastasis, apoptosis induction and/or suppression of neovascularization in combination with other anti-tumor agent or the like and/or surgical therapy or radiotherapy. In addition, the recombinant NK4 protein preparation of the present invention is able to provide a method for the suppression of tumor invasion, growth and metastasis, apoptosis induction and/or suppression of neovascularization in combination with other anti-tumor agent and/or surgical therapy or radiotherapy.

A screening method in accordance with the present invention is
(1) a method (A) for screening a substance which promotes the activity of NK4, characterized in that, the above-mentioned DNA coding for NK4 or NK4 is used
   and, more particularly, it is
(2) a method (B) for screening a substance which promotes the binding activity of NK4 to c-Met/HGF receptor, characterized in that, NK4 and c-Met/HGF receptor are used.

To be more specific, the method (B) for screening in accordance with the present invention provides a method for screening of a substance which promotes the binding activity of NK4 to c-Met/HGF receptor, characterized in that, a binding activity of NK4 to c-Met/HGF receptor in the case of contacting NK4 to c-Met/HGF receptor is measured and compared.

The screening method B may comprise determining the binding activity of NK4 to c-Met/HGF receptor in the presence and in the absence of a given test substance. If the binding activity in the presence of the test substance is higher than the binding activity in the absence of the test substance, the test substance may be selected.

In another embodiment, the screening method B comprises determining the binding activity of NK4 to c-Met/HGF receptor only in the presence of a test substance. The binding activity determined in this way may be compared with a previously determined or known binding activity. The method B can be carried out in vitro.

With regard to the c-Met/HGF receptor (which may be a partial peptide of c-Met/HGF receptor so far as it is able to be bound to NK4) used in the screening method of the present invention, a known c-Met/HGF receptor (Rodrigues, G. A., et al., *Mol. Cell Biol*., **11**, 2962-2970 (1991); and Park, M., et al., *Proc. Natl. Acad. Sci. USA,* **84**, 6379-6383 (1987)) may be used.

The substance which is obtained by using a screening method B of the present invention is a compound which promotes the binding activity of NK4 to c-Met/HGF receptor and its examples are peptides, proteins, synthetic compounds, fermentation products, cell extracts, plant extracts, animal tissue extracts and DNA and etc.

The substance which is obtainedby using a screening method B of the present invention is able to promote the activity of NK4 as a result of promotion of the binding activity of NK4 to c-Met/HGF receptor and, therefore, it is able to be used as a safe and low toxic medicament such as an activity promoter for NK4. Consequently, it can be used as a preventive/therapeutic agent for cancer and a neovascularization suppressor in combination with the above-mentioned DNA coding for NK4, recombinant expression vector containing the said DNA, artificial vector containing the said DNA, transformant which is transformed by the recombinant expression vector containing the said DNA or a medicament containing the recombinant NK4 produced by the transformant.

The substance obtained by the screening method B of the present invention may have anti-cancer action, neovascularization suppressive action, etc. and, in that case, it may be used as an agent for prevention and therapy and as a neovascularization suppressor, for example, for lung cancer, ovarian cancer, pancreatic cancer, stomach cancer, gallbladder cancer, renal cancer, prostatic cancer, breast cancer, esophageal cancer, hepatic cancer, oral cavity cancer, colon cancer, colorectal cancer, uterine cancer, cholangioma, insulinoma, adrenocortical cancer, bladder cancer, testicular cancer, testicular tumor, thyroid cancer, skin cancer, malignant carcinoid tumor, malignant melanoma, osteosarcoma, soft tissue tumor, neuroblastoma, Wilms tumor, retinoblastoma, melanoma and glioma. Among them, ovarian cancer, pancreatic cancer, stomach cancer, gallbladder cancer, renal cancer, prostatic cancer, breast cancer, esophageal cancer, hepatic cancer, oral cavity cancer, colon cancer, colorectal cancer, sarcoma, melanoma or glioma is preferred and, particularly, it can be used as an agent for prevention and therapy and as a neovascularization suppressor for ovarian cancer, pancreatic cancer, stomach cancer and gallbladder cancer.

When the substance obtained by the screening method of the present invention is used as the above-mentioned medicament, it may be made into a preparation by a conventional method and used. For example, the said substance may be used either orally in a form of optionally sugar-coated tablets, capsules, elixir, microcapsules, etc. or parenterally in a form of parenteral injection such as an sterilized solution or suspension with water or other pharmaceutically acceptable liquid. For example, the said substance is made into either an oral preparation together with physiologically acceptable and commonly used carrier, flavor, filler, vehicle, antiseptic agent, stabilizer, binder, etc. or a parenteral injection together with commonly used carrier by a conventional method.

Further, the above-mentioned pharmaceutical composition maybe compounded, for example, with buffer, anesthetizing agent, stabilizer, preservative, antioxidant, etc.

Furthermore, the above-mentioned pharmaceutical composition may be used in combination with an appropriate medicament as a DDS preparation where organ or tissue in which, for example, c-Met/HGF receptor is highly expressed is a specific target.

The pharmaceutical composition prepared as such is safe and has a low toxicity and, therefore, it is able to be administered, for example, to mammals (e.g., human being, rats, mice, rabbits, sheep, swine, cattle, cats, dogs and monkeys).

Dosage of the substance obtained by the screening method of the present invention varies depending upon the object to be administered, object organ, symptom, administering method, etc. and, in the case of administration *per os,* it is about 0.1 to 100 mg, preferably about 1.0 to 50 mg or, more preferably, about 1.0 to 20 mg per day for a cancer patient (body weight being 60 kg). When it is administered parenterally, although the dosage thereof varies depending upon the object to be administered, object organ, symptom, administering method, etc., it is usually preferred in the case of parenteral injection to administer by means of intravenous injection in an amount of about 0.01 to 30 mg, preferably about 0.1 to 20 mg or, more preferably, about 0.1 to 10 mg per day for a cancer patient (body weight being 60 kg). In the case of other animals, it is also possible to administer the dosage which is calculated on the basis of body weight of 60 kg.

### EXAMPLES

As hereunder, Examples of the present invention will be illustrated but the following disclosure shows preferred Examples of the present invention only and does not limit the technical scope of the present invention at all.

### Example 1. Preparation of NK4 cDNA

mRNA was isolated from human MRC-5 fibroblast cells by using Fast Track mRNA isolation kit (Invitrogen) and an RT-PCR (reverse transcription/polymerase chain reaction) is carried out by using the obtained mRNA to gain NK4 cDNA. To be more specific, 0.5 µl (150 ng) of mRNA solution, 5 µl of 10 × RT-PCR solution (500 mM KCl, 100 mM Tris-HCl (pH: 9.0), 1% Triton X-100 and 15 mM MgCl₂), 4 µl of dNTP (2.5 mM), 2 µl of primer: 1 (10 mM), 2 µl of primer 2 (10 mM), 0.5 µl of Taq polymerase (Takara), 0.5 µl of RNasin (Promega), 0.5 µl of reverse transcriptase and 35.2 µl of DEPC-treated H₂O were mixed and subjected to reverse transcription reaction at 42°C for 30 minutes and at 95°C for 5 minutes, a cycle of 94°C for 30 seconds, 55°C for 1 minute and 72°C for 1 minute was repeated for 40 times and further the reaction at 72°C was carried out for 7 minutes to give NK4 cDNA. The NK4 cDNA prepared as such was cloned into PCRII™ vector using TA Cloning Kit (Invitrogen) to give pCRII/NK4.

Sequences of the primer 1 and the primer 2 are as follows.

Primer 1: 5′-CCCGTCCAGCGGTACCATGTGGGTGACC-3′

Primer 2: 5′-TACGGGATGGACTAGTTAGACTATTGTAG-3′

### Example 2. Determination of Base Sequence of NK4 cDNA

The above-prepared NK4 cDNA was made into a sample for the determination of base sequence by Gene Amp PCR System 9600 (Perkin Elmer) using Taq DyeDeoxy™ Terminator Cycle Sequencing Kit (Applied Biosystems) . In the determination of the base sequence, DNA Sequencer Model 377 auto-sequencer manufactured by ABI was used and a base sequence analysis was carried out by a Sanger method using fluorescence-labeled terminator. By the sequencer, two kinds of base sequences - SEQ ID NO: 1 and SEQ ID NO: 2 - were determined.

### Example 3. Construction of Recombinant Adenovirus Expression Vector

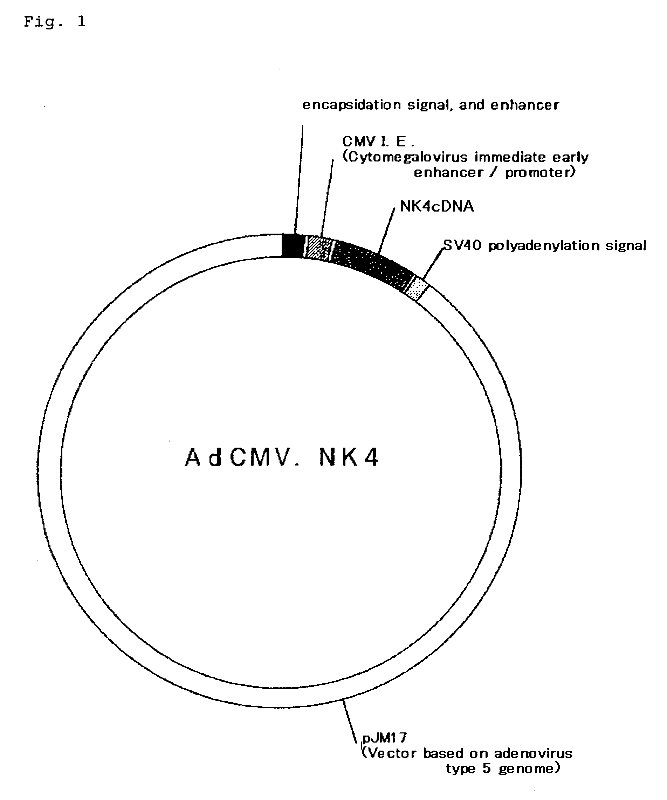

Expression cassette comprising early promoter/enhancer of cytomegalovirus, NK4 cDNA prepared in Example 1 and an SV40 poly(A) signal sequence was inserted into shuttle plasmid vector pSV2+ (Korst, R. J., *Hum. Gene Ther.,* **6,** 277-287 (1995)) to construct a cassette for expression. This cassette for expression and pJM17 (Microbix Biosystems Inc.) comprising genome of adenovirus type 5 which is deficient in E1A and a part of E1B and E3 were co-transfected to HEK 293 cells (human embryonal kidney cells) by a calcium co-precipitation method. Desired virus was selected from the recombinant adenovirus containing expression cassette by the difference in the length of DNA fragments resulted of digestion of virus DNA using restriction enzyme to construct a recombinant adenovirus expression vector AdCMV.NK4 expressing NK4 cDNA. The constructed recombinant adenovirus expression vector is shown in Fig. 1.

### Example 4. Action of AdCMV.NK4 on HGF-Induced Tumor Cell Invasion

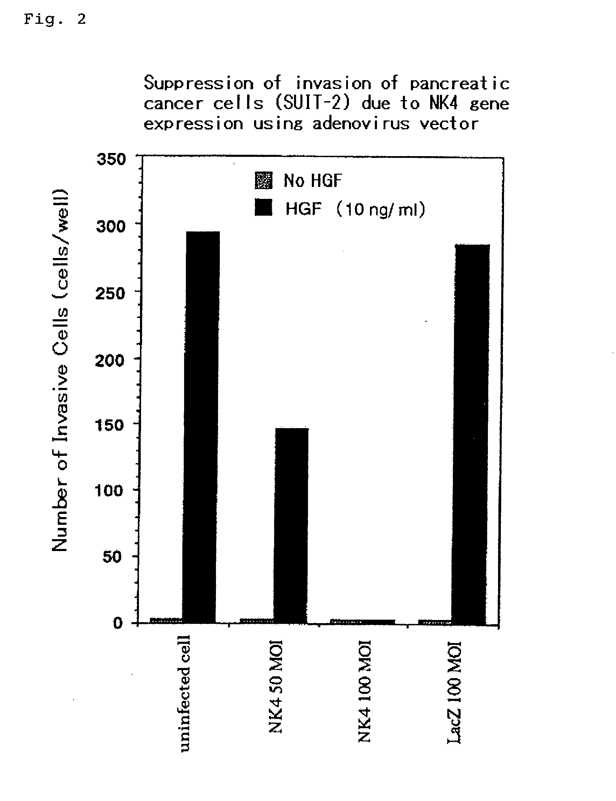

Effect of AdCMV.NK4 on tumor cell invasion induced by HGF was evaluated by a basement membrane invasion model using Matrigel invasion chamber (24 wells). As a control, SUIT-2 cells (human pancreatic cancer cells) and AdcMV.LacZ containing *lacZ* gene of *Escherichia coli* instead of NK4 cDNA were used. Uninfected SUIT-2 cells and infected SUIT-2 cells (human pancreatic cancer cells) with AdCMV.NK4 or AdCMV.LacZ were suspended in an RPMI medium containing 2% of fetal bovine serum (FBS), adjusted to 5 × 10³ cells/well and plated in a transwell of the Matrigel invasion chamber. With regard to an RPMI medium containing 2% of fetal bovine serum (FBS) in a lower cup, that where HGF was present and absent were prepared. After incubation for 24 hours, the cells on the lower side of transwell filter were stained with hematoxylin and eosin. Invasion ability was evaluated under a microscope according to cell numbers which invaded to the lower side of the transwell filter. The result is shown in Fig. 2. As a result, there was almost no cell or only a few cells which invaded to the lower side of the transwell filter in the absence of HGF. On the other hand, in the presence of 10 ng/ml of HGF, untreated SUIT-2 cells which were not infected and SUIT-2 cells which were infected with AdCMV.LacZ invaded strongly. In the meanwhile, invasion of SUIT-2 cells which were infected by AdCMV.NK4 was strongly suppressed in a dependent manner on multiplicity of infection (MOI).

Such an outcome is a result of the fact that HGF promotes the invasion of tumor cells while NK4 produced by AdCMV.NK4 acts as an antagonist to HGF which promotes the invasion of tumor cells, whereby invasion of tumor cells was suppressed.

### Example 5. Tumor Growth Suppression and Neovascularization Suppression by Ad CMV.NK4

Suppressive effect on tumor growth and on neovascularization by AdCMV.NK4 was evaluated using nude mice. As a control group, SUIT-2 cells (human pancreatic cancer cells) and AdCMV.LacZ containing *lacZ* gene of *Escherichia coli* instead of NK4 cDNA were used. SUIT-2 cells (a group to which no Ad was infected), SUIT-2 cells infected with AdCMV.LacZ (AD-LacZ) and SUIT-2 cells infected with AdCMV.NK4 (Ad-NK4) were subcutaneously inoculated to nude mice of 6-week-age (BALB/c nu/nu) (Japan SLC Inc.). AdCMV.Lac Z and AdCMV.NK4 were infected at MOI of 50 (multiplicity of infection). After inoculation of tumor, tumor volume was followed up with a lapse of time. Further, the number of blood vessels were investigated after elapse of 28 days from the tumor inoculation. Tumor volume was calculated by {(width (mm))² × (length (mm))} × 0.52. Incidentally, growth of the tumor was evaluated by measuring the tumor volume.

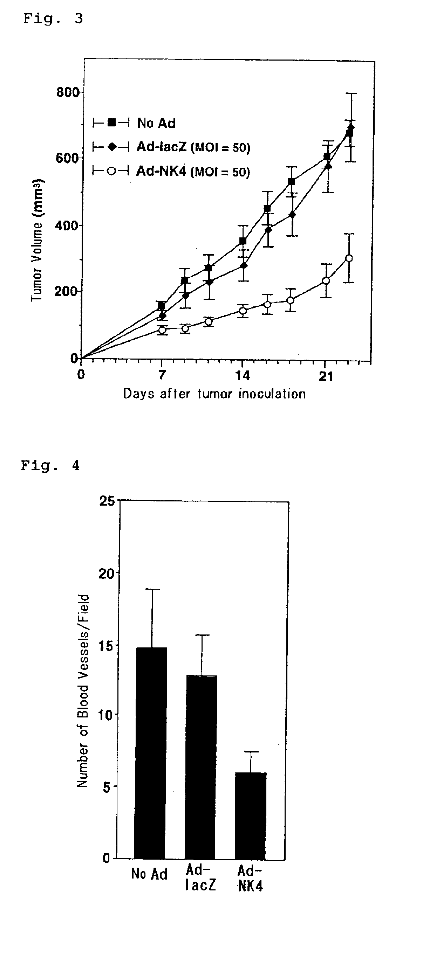

Result of following up of the tumor volume with a lapse of time is shown in Fig. 3. The tumor volume on the 18th day from the inoculation was 539 mm³ for the group to which no Ad was infected, 435 mm³ for AdCMV.LacZ-infected cells and 180 mm³ for AdCMV.NK4-infected cells, which showed that AdCMV.NK4 strongly suppressed the growth of tumor. From the result as such, it was ascertained that NK4 which was produced in tumor cells by AdCMV.NK4 was able to significantly suppress the growth of tumor.

The number of blood vessels after 28 days from the tumor inoculation is shown in Fig. 4. The number of blood vessels around the tumor where AdCMV.Lac Z was infected was 12.8 blood vessels/field, while the number of blood vessels around the tumor where AdCMV.NK4 was infected was 6.1 blood vessels/field (p < 0.05), which showed that AdCMV.NK4 strongly suppressed the neovascularization. From the result as such, it was ascertained that NK4 produced in tumor cells by AdCMV.NK4 was able to significantly suppress the growth of neovascularization.

### Example 6. Tumor Growth Suppression and NK4 Production by NK4 Gene Expression

Tumor growth suppression and NK4 production by expression of NK4 gene were evaluated using nude mice. Expression vector pcDNA3/NK4, which is expressive in mammalian cells, where human

### Example 6. Tumor Growth Suppression and NK4 Production by NK4 Gene Expression

Tumor growth suppression and NK4 production by expression of NK4 gene were evaluated using nude mice. Expression vector pcDNA3/NK4, which is expressive in mammalian cells, where human NK4 cDNA was integrated into expression vector pcDNA 3 was introduced into TMK1 cells (human stomach cancer cells) using DMRIE-C reagent (Life Technologies Inc.). This was incubated for 24 hours in a DMEM/F12 medium containing 10% fetal bovine serum and then incubated in a selective medium consisting of DMEM/F12 containing 300 µg/ml of G 418. TMK 1 cells which constantly produced NK4 were cloned. TMK 1 cells to which no NK4 gene was introduced and TMK 1 cells which constantly produced NK4 were named P and T 11, respectively. P cells and T 11 cells were suspended in 100 µl of a phosphate-buffered physiological saline (PBS) in an amount of 1 × 10⁶ cells each and subcutaneously inoculated into the flanks of nude mice of five-week-age (BALB/c) (Charles River Japan). Tumor volume was calculated by {(width (mm))² × (length (mm))} × 0.52. Growth of tumor was evaluated by measuring the tumor volume.

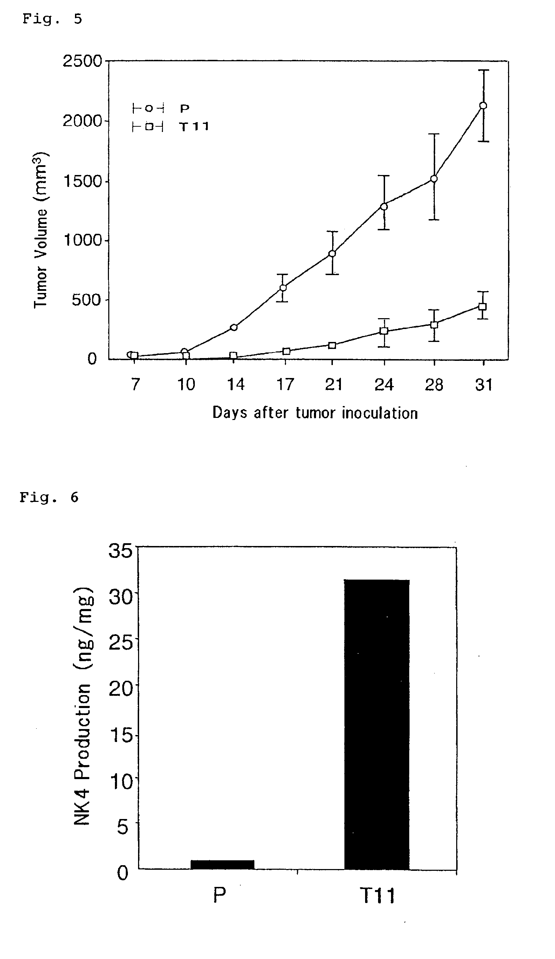

Result of following up of the tumor volume with a lapse of time is shown in Fig. 5. As compared with the mice to which P cells were inoculated, growth of tumor in the mice to which T 11 cells were inoculated was suppressed by 78% (p < 0.01) after 31 days from the inoculation. From the result as such, it was ascertained that growth of tumor was significantly suppressed when NK4 gene was expressed in tumor cells.

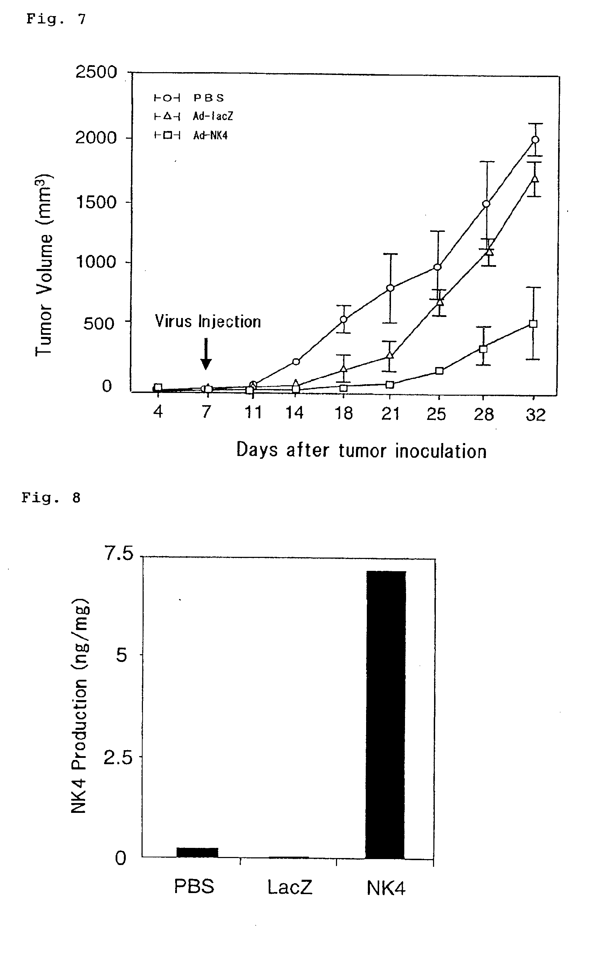

Amount of production of NK4 is shown in Fig. 6. After 31 days from the inoculation, the produced amount of NK4 was measured by the ELISA method using human HGF. Produced amount of NK4 extracted from subcutaneous tumor was 0.06 ng/mg (below the detection limit) in the case of P cells while, in the case of T 11 cells to which pcDNA3/NK4 was transfected, it was 31.4 ng/mg. From the above result, it was ascertained that NK4 was produced in T 11 cells to which pcDNA3/NK4 was transfected.

### Example 7. Tumor Growth Suppression and NK4 Production by AdCMV.NK4

Tumor growth suppression and NK4 production by AdCMV.NK4 were evaluated using nude mice. As a control group, PBS and AdCMV.LacZ containing *lacZ* gene of *Escherichia coli* instead of NK4 cDNA was used. TMK1 cells (human stomach cancer cells) (1 × 10⁶ cells) were suspended in 100 µl of phosphate-buffered physiological saline (PBS) and subcutaneously inoculated into the flanks of nude mice (BALB/c) of five-week-age (Charles River Japan). After 7 days from the inoculation of TMK1 cells, the mice established tumor. 100 µl of PBS alone, or AdCMV.LacZ or AdCMV.NK4 (each in 1 × 10⁹ plaque forming units; pfu) suspended in 100 µl of PBS was administered directly into the tumors of mice for 5 days after 7 days from inoculation of the TMK 1 cells.

Tumor volume was measured. The tumor volume was calculated by {(width (mm))² × (length (mm))} × 0.52. Growth of tumor was evaluated by measuring the tumor volume. Result of following up of the tumor volume with a lapse of time is shown in Fig. 7. After seven days from the inoculation of the TMK 1 cells, diameter of the tumor was about 3 to 5 mm. The tumor where AdCMV.NK4 was infected did not show a tendency of growth until 21st days after the inoculation of tumor. Tumor volume was suppressed by 71% (p < 0.01) as compared with PBS-injected tumor after 31 days from the inoculation of the tumor. From the result as such, it was ascertained that AdcMV.NK4 was able to significantly suppress the tumor growth *in vivo*.

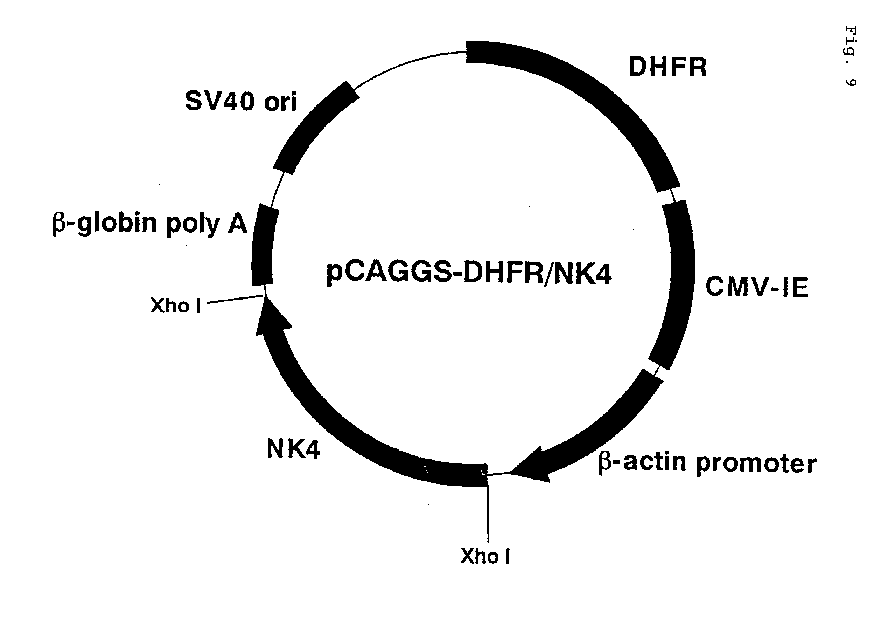

Amount of production of NK4 is shown in Fig. 8. After one week from the inoculation of TMK 1 cells, the produced amount of NK4 was measured by the ELISA method using human HGF. Produced amount of NK4 extracted from subcutaneous tumor was 0.05 ng/mg (below the detection limit) in the case of PBS and 0.01 ng/mg (below the detection limit) in the case of AdCMV.Lac Z while, in the case of AdCMV.NK4, it was 7.36 ng/mg. From the above result, it was ascertained that AdCMV.NK4 produced NK4 *in vivo.*

### Example 8. Construction of Recombinant Expression Vector

NK4 cDNA integrated into pCRII vector prepared in Example 1 was cleaved by restriction enzyme Kpn I/Spe I and the cleaved end was made blunt by treating with T4 DNA polymerase (Takara). The resulting NK4 cDNA fragment was previously treated with a restriction enzyme Xho I, mixed with the expression vector pCAGGS-DHFR for CHO cells where the cleaved end was made blunt and then linked together by T4 DNA ligase to give NK4 expression vectorpCAGGS-DHFR/NK4. The resulting plasmid is shown in Fig. 9. The resulting NK4 expression vector has NK4 cDNA between chicken β-actin promoter and rabbit (3-globin poly(A) signal sequence. In addition, isolation of the transformed cells is possible by DHFR chimera gene where cytomegalovirus early promoter and poly(A) is linked to mouse dihydrofolic acid reductase (DHFR) gene by a signal sequence.

### Example 9. Transformation to Chinese Hamster CHO Cells and Expression Thereof

The above-mentioned vector pCAGGS-DHFR/NK4 for expression in CHO cells were introduced into DHFR deficient cells of Chinese hamster CHO cells by a method of Wigler, et al. *(Cell,* **11,** 233 (1977)). About 30 µg of pCAGGS-DHFR/NK4 plasmid were dissolved in each 240 µl of 0.5M calcium chloride and then 240 µl of 2 × HEPES buffer (pH: 7.1) comprising 20 mM HEPES, 280 mM sodium chloride and 1.5 mM sodium phosphate was added thereto with stirring. Stirring was continued at room temperature for 30 minutes to form a co-precipitate of the plasmid with calcium phosphate. After that, 5 × 10⁵ CHO cells were incubated at 37°C for 24 hours in the presence of 5% CO₂ using an α-MEM medium (Flow Laboratory) containing 10% fetal bovine serum (Gibco) and 1% glutamine. After replacement of the medium, the co-precipitate of plasmid with calcium phosphate was added thereto and the mixture was allowed to stand at room temperature for 20 minutes. After that was further incubated for 4 hours at 37°C, the medium was removed, then 1 × HEPES buffer to which 15% glycerol were added was added and the mixture was allowed to stand at room temperature for 5 minutes. After the cells were washed with the medium, the medium was replaced and incubation was further carried out at 37°C for 7 days to give a transformed cells. The resulting cell strain was repeatedly subjected to a subculture in the said medium using an α-MEM medium (Flow Laboratory) containing neither ribonucleoside nor deoxyribonucleoside and containing 10% dialyzed fetal bovine serum (Gibco) and 2% glutamine where methotrexate concentrations were successively changed to 100 nM, 250 nM, 500 nM, 750 nM, 1 µM and 2 µM so as to give a stable strain producing high amount of NK4. The resulting NK4-productive recombinant cells were subjected to a clone selection to give a stable NK4-productive strain.

### Example 10. Purification of Recombinant NK4 from Supernatant of Transformed CHO Cell Culture

The NK4-producing Chinese hamster CHO recombinant cell strain obtained in the above Example 9 was incubated in an α-MEM medium (Flow Laboratory) containing neither ribonucleoside nor deoxyribonucleoside but containing 10% fetal bovine serum (Gibco), 1% glutamine and 2 µM of methotrexate, and recombinant NK4 was purified from the supernatant of culture.

### 1) Heparin affinity chromatography

Tween 80 was added to 12 liters of culture medium of NK4-productive Chinese hamster CHO recombinant cell strain so as to make the final concentration 0.01% and the mixture was filtered through a Sterivex HV filter (Nippon Millipore) . This was added to heparin-Sepharose CL-6B (manufactured by Pharmacia; column volume: 50 ml) which was equilibrated with a buffer A (20 M citrate-NaOH and 0.01% Tween 80; pH: 6.5) containing 0.15 M sodium chloride. After washed with a buffer A containing 0.5 M sodium chloride, peak fractions eluted with a linear concentration gradient from 0.5 M to 2.5 M sodium chloride were collected to give a heparin eluate A.

### 2) Anion-exchange chromatography

Heparin eluate A was dialyzed for three times against 100-fold volume of buffer B (20 mM Tris-HCl and 0.01% Tween 80; pH: 8.0) and added to DEAE-Sepharose (made of Pharmacia; column volume: 40 ml) equilibrated with a buffer B. After washed with a buffer B, peak fractions eluted with a buffer B containing 1M sodium chloride were collected to give a DEAE eluate.

### 3) Heparin affinity chromatography (second run)

The DEAE eluate was dialyzed for three times against 100-fold volume of buffer A and added to heparin-Sepharose CL-6B (made of Pharmacia; column volume: 50 ml) equilibrated with a buffer A containing 0.15 M sodium chloride. After washed with a buffer A containing 0.3 M sodium chloride, the absorbed thing was eluted by means of a linear concentration gradient of from 0.3 M to 2.5 M sodium chloride. The peak fractions of NK4 was collected to give a heparin eluate B. Yield of the purified recombinant NK4 was about 12 mg and the recovery ratio from the supernatant of culture liquid was about 50%.

### 4) SDS-Polyacrylamide electrophoresis

The recombinant NK4 with and without being reduced by 2-mercaptoethanol was subjected to an SDS-polyacrylamide electrophoresis. The purified recombinant NK4 under the non-reduced condition (2-ME(-)) was about 50 kDa and, under the reduced condition (2-ME(+)), it was about 67 kDa.

### Example 11. Suppression of growth of tumor by recombinant NK4

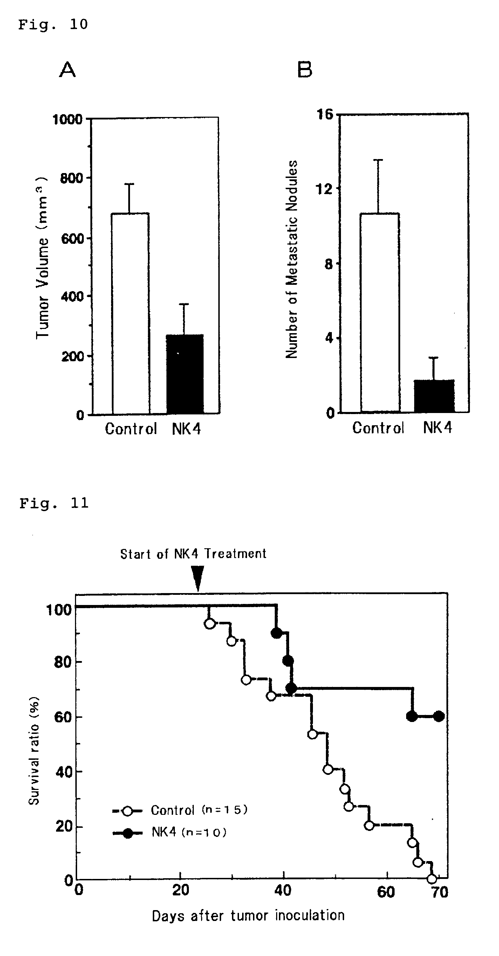

Effect of suppression on tumor growth by the recombinant NK4 was investigated. Tumor volume was calculated by {(width (mm))² × (length (mm))} × 0.52. Growth of tumor was evaluated by measuring the tumor volume. Result of measurement of tumor volume is shown in Fig. 10A. A suspension (50 µl) of SUIT-2 cells (human pancreatic cancer cells) (2 × 10⁷ cells/ml) was inoculated into pancreas of nude mice (BALB/c nu/nu) (Japan SLC Inc.) of six-week-age. The recombinant NK4 purified in Example 10 being mixed with physiological saline was intraperitoneally administered (1.5 mg/kg/day) into the above mentioned mice for 25 days twice daily. As a control, the same dosage (1.5 mg/kg/day) of BSA was intraperitoneally administered for 25 days twice daily. As compared with the control, administration of the recombinant NK4 suppressed the tumor growth by 60.7% (p < 0.05). In the control group, invasion to the surrounding of the spleen and further to epithelial membrane of pancreas was noted while, in the group to which NK4 was administered, invasion to the surrounding of spleen and to the epithelial membrane was not noted. As a result, it was ascertained that the recombinant NK4 acted as an antagonist to HGF, suppressed the invasion of tumor cells and suppressed the growth of tumor.

### Example 12. Suppression of metastatic nodule

Metastasis-suppressive effect by the recombinant NK4 was investigated. The metastasis-suppressive effect was evaluated by counting the metastatic nodule on mesentery and peritoneal wall. Counting of metastatic nodule number was carried out by observing the metastatic nodule of 1 mm or longer by naked eye. The result of counting of metastatic nodule is shown in Fig. 10B. A suspension (50 µl) of SUIT-2 cells (human pancreatic cancer cells) (2 × 10⁷ cells/ml) was inoculated into pancreas of nude mice (BALB/c nu/nu) (Japan SLC Inc.) of six-week-age. The above-mentioned recombinant NK4 mixed with a physiological saline was intraperitoneally administered (1.5 mg/kg/day) into the above mentioned mice for 25 days twice daily. As a control, the same amount of BSA (1.5 mg/kg/day) was intraperitoneally administered for 25 days twice daily. As compared with the control (BSA), administration of the recombinant NK4 suppressed the numbers of metastatic nodule on mesentery, diaphragma and peritoneum of nude mice by 84.1% (p<0.05). As a result, it was ascertained that the recombinant NK4 suppressed the metastasis of tumor cells.

### Example 13. Effect on survival ratio by administration of recombinant NK4

Effect of administration of the recombinant NK4 on terminal cancer was evaluated. Result of evaluating the survival ratio is shown in Fig. 11. Administration of NK4 was started on the 24th day from inoculation of 50 µl of a suspension of SUIT-2 cells (human pancreatic cancer cells) (2 × 10⁷ cells/ml) to nude mice (BALB/c nu/nu) of six weeks age. In control mice (n = 15) to which no NK4 was administered, death started on the 26th day from the inoculation of the SUIT-2 cells and, within the 69th day, all mice were dead. On the contrary, in the mice (n = 10) to which NK4 was administered, although 4 were dead in 65 days from the inoculation of the SUIT-2 cells, 6 were still alive even after 70 days. From the result, it was ascertained that administration of the recombinant NK4 has a life-prolonging effect even in the stage of terminal cancer.

### Example 14. Suppression of Growth of Melanoma by Gene Therapy with Ad-CMV.NK4

Suppressive effect of Ad-CMV.NK4 on growth of mouse melanoma was examined in mice. B16F10 mouse melanoma cells (10⁶ cells) were subcutaneously inoculated into the back of 6-10 weeks old male C57BL6J mice (Japan SLC Inc.). Twenty-four hours after tumor inoculation, mice were injected with PBS (phosphate-buffered saline) or 10⁹ pfu (plaque forming unit) Ad-CMV.LacZ or Ad-CMV.NK4. The size of tumors was measured using a dial caliper, and the volume of tumor was calculated using the formula width (mm)² x length (mm) x 0.52.

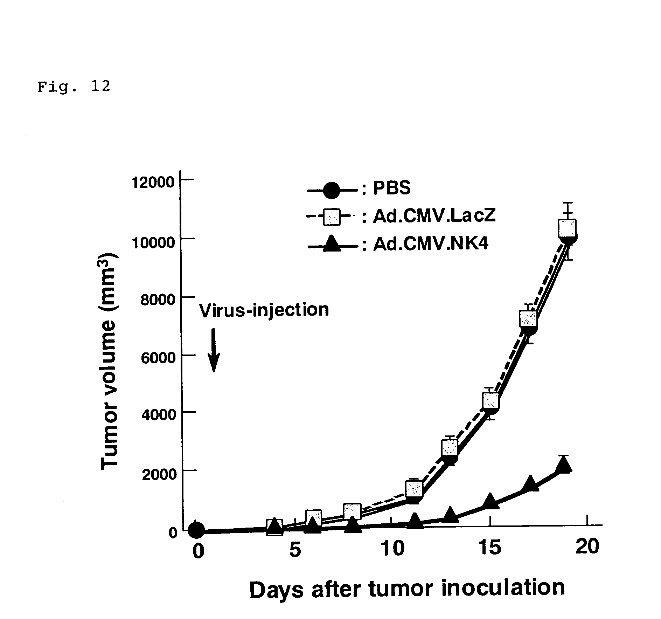

Result of following up of the tumor volume with a lapse of time is shown in Fig. 12. Tumors developed rapidly in mice injected with PBS or Ad-CMV.LacZ. Tumors grew at a much slower rate in mice infected with Ad-CMV.NK4. On the 19th day after tumor inoculation, the tumor volume in mice injected with Ad-CMV.NK4 was suppressed to about 20% of that seen in control mice injected with PBS or Ad-CMV.LacZ. The result indicates that treatment with Ad-CMV.NK4 suppressed growth of melanoma in mice.

### Example 15. Suppression of Lung Metastasis of Melanoma by Gene Therapy with Ad-CMV.NK4

Suppressive effect of Ad-CMV.NK4 on lung metastasis of mouse melanoma was examined in mice. B16F10 mouse melanoma cells (10⁶ cells) were injected into the tail vein of 6-8 weeks old male C57BL6J mice (Japan SLC Inc.). Twenty-four hours after tumor cell injection, mice were injected with PBS or 10⁹ pfu (plaque forming unit) Ad-CMV.LacZ or Ad-CMV.NK4. On 21st day after tumor cell injection, the number of metastatic nodules in the lung was counted microscopically.

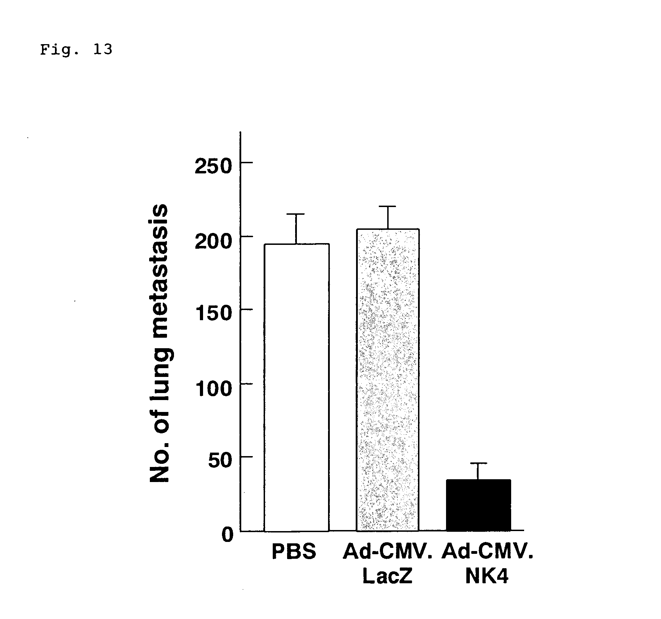

Result of effect of Ad-CMV.NK4 on lung metastasis of mouse melanoma is shown in Fig. 13. In control mice injected with PBS or Ad-CMV.LacZ, the number of lung metastasis reached about 200. In contrast, the number of lung metastasis remained about 35. The result indicates that treatment with Ad-CMV.NK4 inhibited metastasis of melanoma in mice.

### Example 16. Construction of Naked Expression Plasmid for Administration of Human NK4

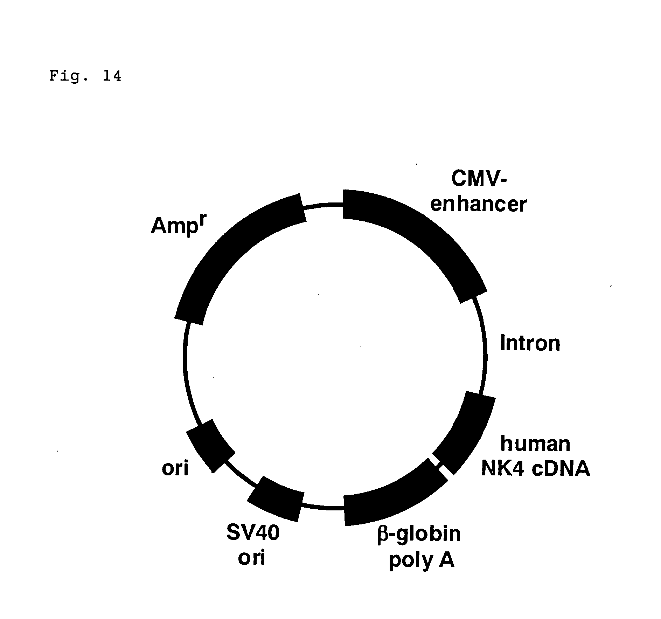

Human NK4 cDNA integrated into pCRII vector prepared in Example 1 was cleaved by restriction enzyme Kpn I/Spe I and the cleaved end was made blunt by treating with T4 DNA polymerase (Takara). pCAG plasmid (Niwa et al., Gene, 108: 193-200, 1991) was cleaved by restriction enzyme Xho I and the cleaved end was made blunt by treating with T4 DNA polymerase. The NK4 cDNA fragment was linked together by T4 DNA ligase to give expression plasmid pCAG-NK4 for mammalian cells. The resulting plasmid is shown in Fig.14. The expression plasmid has human NK4 cDNA between chicken β-actin promoter and rabbit β-globin poly(A) signal sequence.

### Example 17. Suppression of Growth of Colon Cancer by Gene Therapy with pCAG-NK4 Plasmid

Suppressive effect of gene expression of NK4 using naked plasmid (pCAG-NK4) administration on growth of colon cancer was examined in mice. MC-38 mouse colon cancer cells (10⁶ cells) were subcutaneously inoculated into the back of 7 weeks old male C57BL6J mice (Japan SLC Inc.). Expression plasmids (pCAG-empty or pCAG-NK4 plasmid) containing 10 µg DNA were dissolved in 1.6 ml saline. Mice were intravenously injected from the tail vein with pCAG-empty plasmid (for control) or pCAG-NK4 plasmid at 10 µg DNA/mouse on the 5th, 12th, 19th, and 26th day after tumor inoculation. The size of tumors was measured using a dial caliper, and the volume of tumor was calculated using the formula width (mm)² x length (mm) x 0.52.

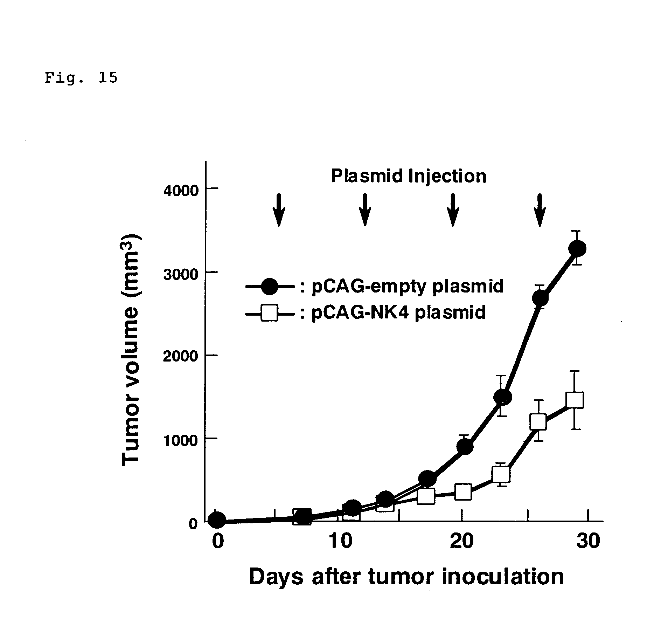

Result of following up of the tumor volume with a lapse of time is shown in Fig.15. Tumors developed rapidly in mice injected with pCAG empty plasmid. Tumors grew at a slower rate in mice infected with pCAG-NK4 plasmid. On the 30th day after tumor inoculation, the tumor volume in mice injected with pCAG-NK4 plasmid was suppressed to about 43% of that seen in control mice injected with pCAG empty plasmid. The result indicates that NK4 gene therapy with pCAG-NK4 plasmid suppressed growth of colon cancer in mice.

### Example 18. Suppression of Liver Metastasis of Colon Cancer by Gene Therapy with pCAG-NK4 Plasmid

Suppressive effect of gene expression of NK4 using naked plasmid (pCAG-NK4) administration on liver metastasis of colon cancer was examined in mice. Expression plasmids (pCAG-empty or pCAG-NK4 plasmid) containing 10 µg DNA were dissolved in 1.6 ml saline. Mice were intravenously injected from the tail vein with pCAG-empty plasmid (for control) or pCAG-NK4 plasmid at 10 µg DNA/mouse. Twenty-four hours after plasmid injection, MC-38 mouse colon cancer cells (10⁶ cells) were inoculated into the spleen of 7 weeks old male C57BL6J mice (Japan SLC Inc.). On 21st day after plasmid injection, the number of metastatic nodules in the liver was counted microscopically.

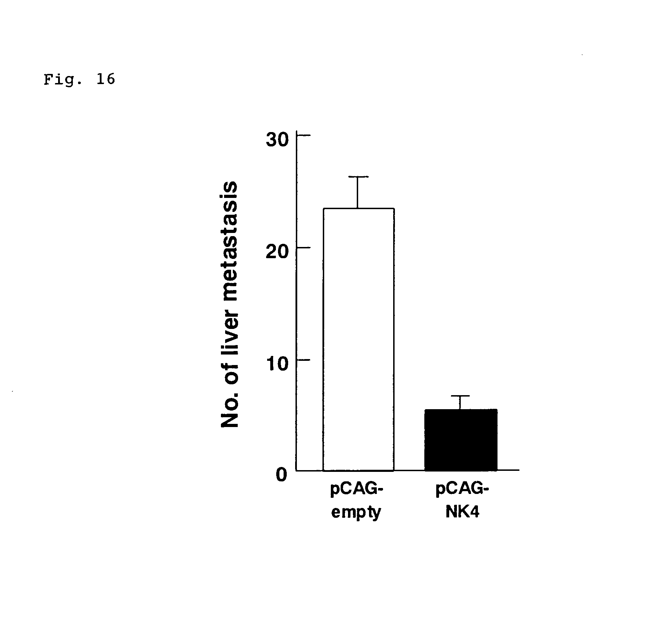

Result of effect of gene therapy by naked plasmid (pCAG-NK4) administration on liver metastasis of mouse colon cancer is shown in Fig. 16. In control mice injected with pCAG empty plasmid, the number of liver metastasis reached 23 in the mean number. In contrast, the number of liver metastasis remained 7 in the mean number. The result indicates that gene therapy with pCAG-NK4 plasmid inhibited metastasis of colon cancer in mice.

### Preparation Example 1

A solution where 1 g of the recombinant NK4 prepared in Example 10, 1 g of mannitol and 10 mg of Polysorbate 80 were contained in 100 ml of physiological saline was aseptically prepared, then each 1 ml thereof was placed in a vial, after that freeze-dried and tightly sealed whereupon the medicament of the present invention was prepared as a freeze-dried agent.

### Preparation Example 2

An aqueous solution containing 1 g of the recombinant NK4 prepared in Example 10 and 100 mg of human serum albumin was aseptically blended with 100 ml of 0.02 M phosphate buffer (containing 0.15 M NaCl and 0.01% of Polysorbate 80; pH: 7.4) and an aliquot of 1 ml thereof was placed in a vial. After that, the liquid in each of the vials was freeze-dried and tightly sealed whereupon the medicament of the present invention was prepared as a freeze-dried agent.

### Preparation Example 3

An aqueous solution containing 1 g of the recombinant NK4 prepared in Example 10, 2 mg of sorbitol, 2 mg of glycine and 10 mg of Polysorbate 80 in 100 ml of distilled water for injection was aseptically prepared and an aliquot of 1 ml thereof was placed in a vial, freeze-dried and tightly sealed whereupon the medicament of the present invention was prepared as a freeze-dried agent.

### INDUSTRIAL APPLICABILITY

By using a medicament containing DNA of the present invention coding for NK4, recombinant expression vector containing the said DNA, artificial vector containing the said DNA or transformant which is transformed by the recombinant expression vector containing the said DNA, it is able to achieve a high anti-tumor property due to suppression of tumor invasion, growth and metastasis, apoptosis induction and/or suppression of neovascularization to human being or animals such as mice, monkeys, dogs, cats, horses and swine whereby it is useful for the prevention and therapy of cancer diseases and is also useful for the prevention and therapy of diseases caused by neovascularization.

Further, when a medicament containing DNA of the present invention coding for NK4, recombinant expression vector containing the said DNA, artificial vector containing the said DNA or transformant which is transformed by the recombinant expression vector containing the said DNA is administered into tumor tissue or tumor cell, the NK4 is able to be made present in high concentrations whereby the therapeutic effect is promoted. On the other hand, by using a medicament containing DNA coding for NK4, recombinant expression vector containing the said DNA, artificial vector containing the said DNA or transformant which is transformed by the recombinant expression vector containing the said DNA, the NK4 which is produced in tissues or cells in body acts on other tissues or cells whereby it is able to achieve an effect of suppressing tumor invasion, growth and metastasis, apoptosis induction and/or suppression of neovascularization not only on primary tumor but also on metastatic cancer.

Furthermore, since NK4 is a fragment of HGF existing in body, it is able to suppress the immunorejection caused by introduced DNA and produced protein, therefore it may be said that its safety is high.

Accordingly, the present invention is able to greatly contribute to gene therapy for the cancer and/or diseases caused by neovascularization.

Still further, as a result of introducing the recombinant expression vector containing DNA coding for NK4 into host, it is now possible to stably and easily produce the recombinant NK4 having a biological activity in volume, which has been difficult to do so up to now and, as a result of suppression of tumor invasion, growth and metastasis, apoptosis induction and/or suppression of neovascularization by the recombinant NK4, the product is able to greatly contribute as a preventive and therapeutic agent for diseases caused by cancer and/or neovascularization and as a preventive agent for metastasis of cancer.

Furthermore, a method for screening a substance which promotes the binding activity between NK4 and c-Met/HGF receptor according to the present invention, a substance which is obtained by the said screening method or a medicament containing the substance obtained by the said screening is able to make the effect of an anti-cancer agent and a neovascularization suppressor containing the recombinant NK4 of the present invention or DNA coding therefor, a recombinant expression vector containing the said DNA, an artificial vector containing the said DNA, a transformant which is transformed by a recombinant expression vector containing the said DNA and the recombinant NK4 produced by the said transformant more useful.

## Claims

1. **DNA which contains**
(a) a base sequence as shown in SEQ ID NO:1;
(b) a base sequence as shown in SEQ ID NO:2; or
(c) DNA hybridizing to the DNA as defined in (a) or (b) under stringent conditions.

2. The DNA according to claim 1, wherein the DNA contains a base sequence as shown in SEQ ID NO:1 or SEQ ID NO:2.

3. A recombinant expression vector containing the DNA according to claim 1 or 2.

4. The recombinant expression vector according to claim 3, wherein the expression vector is adeno-associated virus (AAV), retrovirus, poxvirus, herpes virus, herpes simplex virus, lentivirus (HIV), Sendai virus, Epstein-Barr virus (EBV), vaccinia virus, poliovirus, Sindbis virus, SV 40 or a plasmid.

5. A liposome containing the DNA according to claim 1 or 2 or the recombinant expression vector according to claim 3 or 4.

6. A transformant which is transformed by the recombinant expression vector according to claim 3 or 4.

7. The transformant according to claim 6, wherein the host is bacterium, yeast, plant cell or mammalian cell.

8. A process for the production of a protein encoded by the DNA according to claim 1, **characterized in that** the transformant according to claim 6 or 7 is incubated and the protein encoded by the DNA according to claim 1 is produced.

9. A recombinant protein obtainable by the process according to claim 8.

10. A medicament containing the DNA according to claim 1 or 2, the recombinant expression vector according to claim 3 or 4, the liposome according to claim 5, the transformant according to claim 6 or 7 or the recombinant protein according to claim 9.

11. The medicament according to claim 10, wherein it is an anti-cancer agent.

12. The medicament according to claim 10 or 11, wherein it is an agent for the prevention and/or therapy of ovarian cancer, pancreatic cancer, stomach cancer, gallbladder cancer, renal cancer, prostatic cancer, breast cancer, esophageal cancer, hepatic cancer, oral cavity cancer, colon cancer, colorectal cancer, sarcoma, glioma or melanoma.

13. The medicament according to claim 10, wherein it is a neovascularization suppressor.

14. The medicament according to any one of claims 10 to 13 wherein the DNA according to claim 1 or 2, the recombinant expression vector according to claim 3 or 4, the liposome according to claim 5, the transformant according to claim 6 or 7 or the recombinant protein according to claim 9 is combined with at least one other anti-tumor agent.

15. The use of the DNA according to claim 1 or 2, the recombinant expression vector according to claim 3 or 4, the liposome according to claim 5, the transformant according to claim 6 or 7 or the recombinant protein according to claim 9 for the manufacture of a medicament for the prevention and/or treatment of cancerous disease in mammals.

16. The use according to claim 15, wherein an effective dose of at least one other anti-tumor agent is further administered to the mammal.

17. The use of the DNA according to claim 1 or 2, the recombinant expression vector according to claim 3 or 4, the liposome according to claim 5, the transformant according to claim 6 or 7 or the recombinant protein according to claim 9 for the manufacture of a medicament for the prevention of metastasis of cancer in mammals.

18. The use of the DNA according to claim 1 or 2, the recombinant expression vector according to claim 3 or 4, the liposome according to claim 5, the transformant according to claim 6 or 7 or the recombinant protein according to claim 9 for the manufacture of a medicament for the suppression of neovascularization in mammals.

19. A method for the screening of a substance which promotes the binding activity of the protein encoded by the DNA according to claim 1 to a c-Met/HGF receptor, **characterized in that** the protein encoded by the DNA according to claim 1 and a c-Met/HGF receptor are used.

20. A substance which promotes the binding activity of the protein encoded by the DNA according to claim 1 to a c-Met/HGF receptor obtained by the method according to claim 19.

21. A medicament comprising the substance according to claim 20.
